(19) Europäisches Patentamt
European Patent Office
Office européen des brevets

(11) Veröffentlichungsnummer: **0 176 956 B1**

(12) # EUROPÄISCHE PATENTSCHRIFT

(45) Veröffentlichungstag der Patentschrift: **15.06.94**

(51) Int. Cl.5: **C07D 211/90**, C07D 401/14, C07D 409/14, C07D 405/14, C07D 413/14, C07D 417/14, A61K 31/44, //C07D211/14, C07D211/22

(21) Anmeldenummer: **85112196.2**

(22) Anmeldetag: **26.09.85**

Die Akte enthält technische Angaben, die nach dem Eingang der Anmeldung eingereicht wurden und die nicht in dieser Patentschrift enthalten sind.

(54) **Neue Diarylverbindungen.**

(30) Priorität: **28.09.84 CH 4652/84**
**28.09.84 CH 4653/84**

(43) Veröffentlichungstag der Anmeldung:
**09.04.86 Patentblatt 86/15**

(45) Bekanntmachung des Hinweises auf die Patenterteilung:
**15.06.94 Patentblatt 94/24**

(84) Benannte Vertragsstaaten:
**AT BE CH DE FR GB IT LI LU NL SE**

(56) Entgegenhaltungen:
**EP-A- 0 088 903**
**EP-A- 0 094 159**
**EP-A- 0 106 276**

(73) Patentinhaber: **Byk Gulden Lomberg Chemische Fabrik GmbH**
**Postfach 10 03 10**
**D-78403 Konstanz(DE)**

(72) Erfinder: **Amschler, Hermann, Dr.**
**Hohenhewenstrasse 19**
**D-7760 Radolfzell(DE)**
Erfinder: **Beume, Rolf, Dr.**
**Birnaublick 10**
**D-7750 Konstanz(DE)**
Erfinder: **Eistetter, Klaus, Dr.**
**Säntisblick 7**
**D-7750 Konstanz 19(DE)**
Erfinder: **Eltze, Manfrid, Dr.**
**Schützenstrasse 20**
**D-7750 Konstanz(DE)**
Erfinder: **Flockerzi, Dieter, Dr.**
**Ackerweg 26**
**D-7753 Allensbach(DE)**
Erfinder: **Kilian, Ulrich, Dr.**
**Stettinerstrasse 22**
**D-7750 Konstanz(DE)**
Erfinder: **Klemm, Kurt, Prof. Dr.**
**Im Weinberg 2**
**D-7753 Allensbach(DE)**
Erfinder: **Kolassa, Norbert, Prof. Dr.**
**Lerchenweg 12**
**D-7750 Konstanz(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 176 956 B1

Erfinder: **Sanders, Karl, Dr.**
**Felchengang 23**
**D-7750 Konstanz(DE)**
Erfinder: **Ulrich, Wolf-Rüdiger, Dr.**
**Brandesstrasse 14**
**D-7750 Konstanz(DE)**

## Beschreibung

Anwendungsgebiet der Erfindung

Die Erfindung betrifft neue Diarylverbindungen, Verfahren zu ihrer Herstellung, ihre Verwendung und sie enthaltende Arzneimittel. Die erfindungsgemäßen Verbindungen werden in der pharmazeutischen Industrie zur Herstellung von Arzneimitteln eingesetzt.

Bekannter technischer Hintergrund

Es ist bekannt, daß bestimmte, auf verschiedene Weise substituierte 1,4-Dihydropyridinderivate pharmakologisch nützliche Eigenschaften aufweisen. Beispielsweise seien die europäischen Patentanmeldungen 88 903, 94 159 und 106 276 genannt. Überraschenderweise wurde nun gefunden, daß die unten näher beschriebenen neuen Verbindungen, die im Unterschied zu den Verbindungen des Standes der Technik einen in 4-Position zweifach substituierten Piperidinring tragen, besonders interessante pharmakologische Eigenschaften aufweisen, durch die sie sich von den Verbindungen des Standes der Technik in vorteilhafter Weise unterscheiden.

Beschreibung der Erfindung

Gegenstand der Erfindung sind neue Diarylverbindungen der Formel I

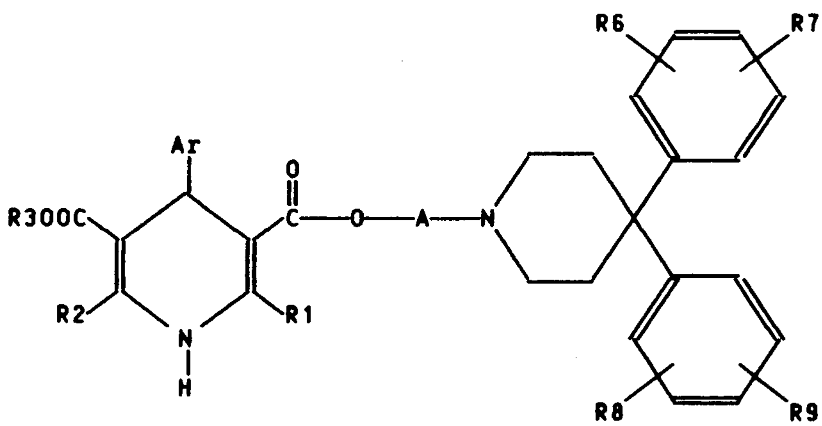

(I),

worin

Ar einen Cyclus der Formel

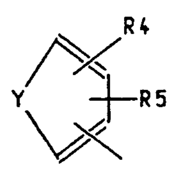

darstellt, in dem Y Sauerstoff (O) Schwefel (S), Vinylen (-CH = CH-), Azomethin (-CH = N-) oder eine Gruppe der Formel

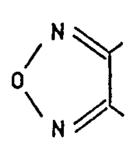

oder

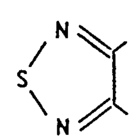

| | |
|---|---|
| | bedeutet, |
| R1, R2 und R3 | gleich oder verschieden sind und Wasserstoff, $C_1$-$C_6$-alkyl oder $C_3$-$C_7$-alkoxyalkyl bedeuten, |
| R4 und R5 | gleich oder verschieden sind und Wasserstoff, Hydroxy, Halogen, Nitro, Cyano, Trifluormethyl, $C_1$-$C_4$-alkyl, $C_1$-$C_4$-alkoxy, ganz oder teilweise durch Fluor substituiertes $C_1$-$C_4$-alkoxy, $C_1$-$C_4$-alkoxycarbonyl, $C_2$-$C_5$-acyl, Amino oder Mono- oder Di-$C_1$-$C_4$-alkylamino bedeuten, |
| R6, R7, R8 und R9 | gleich oder verschieden sind und Wasserstoff, Hydroxy, Halogen, $C_1$-$C_4$-alkyl, $C_1$-$C_4$-alkoxy, Amino, Mono- oder Di-$C_1$-$C_4$-alkylamino oder ganz oder teilweise durch Fluor substituiertes $C_1$-$C_4$-alkoxy bedeuten, |
| A | geradkettiges oder verzweigtes $C_2$-$C_5$-alkylen bedeutet |

und ihre Salze.

$C_1$-$C_6$-alkyl ist geradkettig oder verzweigt und bedeutet beispielsweise einen Hexyl-, Neopentyl-, Isopentyl-, Butyl-, i-Butyl-, sec. -Butyl-, t-Butyl-, Propyl-, Isopropyl- oder insbesondere Ethyl- oder Methylrest.

$C_3$-$C_7$-alkoxyalkyl steht beispielsweise für einen Methoxyethyl-, Ethoxyethyl-, Propoxyethyl-, Isopropoxyethyl-, Butoxyethyl-, Methoxypropyl-, 2-Methoxy-1-methylethyl- oder 2-Ethoxy-1-methylethylrest.

Halogen bedeutet Brom und insbesondere Fluor und Chlor.

$C_1$-$C_4$-alkyl ist geradkettig oder verzweigt und bedeutet beispielsweise einen Butyl-, i-Butyl-, sec.-Butyl-, t-Butyl-, Propyl-, Isopropyl-, Ethyl- oder insbesondere Methylrest.

$C_1$-$C_4$-alkoxy enthält neben dem Sauerstoffatom einen der vorstehend genannten $C_1$-$C_4$-alkylreste. Bevorzugt ist der Methoxyrest.

Ganz oder teilweise durch Fluor substituiertes $C_1$-$C_4$-alkoxy ist beispielsweise 1,1,2,2-Tetrafluorethoxy, Trifluormethoxy, 2,2,2-Trifluorethoxy oder Difluormethoxy.

$C_1$-$C_4$-alkoxycarbonyl enthält neben der Carbonylgruppe einen der vorstehend genannten $C_1$-$C_4$-alkoxyreste. Bevorzugt sind der Methoxycarbonyl- und der Ethoxycarbonylrest.

$C_2$-$C_5$-acyl enthält neben der Carbonylgruppe einen der vorstehend genannten $C_1$-$C_4$-alkylreste. Bevorzugt ist der Acetylrest.

Mono- oder Di-$C_1$-$C_4$-alkylamino enthält neben dem Stickstoffatom einen oder zwei der vorstehend genannten $C_1$-$C_4$-alkylreste. Bevorzugt ist Di-$C_1$-$C_4$-alkylamino, und hier insbesondere Dimethyl-, Diethyl- oder Diisopropylamino.

Geradkettiges oder verzweigtes $C_2$-$C_5$-alkylen ist beispielsweise Tetramethylen, 1,2-Dimethylethylen, 1,1-Dimethylethylen, 2,2-Dimethylethylen, Isopropyliden, 1-Methylethylen, 2-Ethylpropylen und insbesondere Ethylen oder Propylen.

Als Salze kommen alle Salze mit Säuren in Betracht. Besonders erwähnt seien die pharmakologisch verträglichen Salze der in der pharmazeutischen Industrie üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 2-Hydroxy-3-naphthoat, 3-Hydroxy-2-naphthoat oder Mesilat, aber auch Salze mit Bumetanid, Furosemid, Azosemid, Galosemid, Besunid, Piretanid, Etacrynsäure, Tienilinsäure oder 4-Chlor-sulfamoyl-benzoesäure.

Hervorzuhebende Reste Ar sind der Phenyl-, 3-Fluorphenyl-,2-Chlorphenyl-, 3-Chlorphenyl-, 4-Chlorphenyl-, 2,3-Dichlorphenyl-, 2-Cyanophenyl-,3-Cyanophenyl-, 2-Methoxyphenyl-, 3-Methoxyphenyl-, 2-Trifluormethylphenyl-, 3-Trifluormethylphenyl-, 3-(1,1,2,2-Tetrafluorethoxy)-phenyl-, 2-Difluormethoxyphenyl-, 3-Difluormethoxyphenyl-, 2-Tolyl-, 3-Tolyl-, 4-Tolyl-, 2-Pyridyl-, 3-Pyridyl-, 2,1,3-Benzoxdiazol-4-yl-, 5-Methyl-2-thienyl- und insbesondere der 2-Nitrophenyl- und der 3-Nitrophenylrest.

Ausgestaltungen der Erfindung sowie bevorzugte und besonders bevorzugte Ausführungsformen sind in den Ansprüchen aufgeführt.

Als erfindungsgemäße Verbindungen seien beispielsweise genannt:

1,4-Dihydro-2,6-dimethyl-4-(3-nitrophenyl)-pyridin-3,5-dicarbonsäure-3-ethyl-5-[2-(4,4-diphenylpiperidyl-1)-ethyl]-ester,

1,4-Dihydro-2,6-dimethyl-4-(3-nitrophenyl)-pyridin-3,5-dicarbonsäure-3-(2-methoxyethyl)-5-[3-(4,4-diphenylpiperidyl-1)-propyl]-ester,

1,4-Dihydro-2,6-dimethyl-4-(3-nitrophenyl)-pyridin-3,5-dicarbonsäure-3-methyl-5-[4-(4,4-diphenylpiperidyl-1]-

butyl]-ester,
1,4-Dihydro-2,6-dimethyl-4-(2-nitrophenyl)-pyridin-3,5-dicarbonsäure-3-methyl-5-[2-(4,4-diphenylpiperidyl-1)-ethyl]-ester,
1,4-Dihydro-2,6-diethyl-4-(3-nitrophenyl)-pyridin-3,5-dicarbonsäure-3-methyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(propyl-2)-5-[2-(4,4-diphenylpiperidyl-1)-ethyl]-ester,
1,4-Dihydro-2,6 -dimethyl-4-(3-nitrophenyl)-pyridin-3,5-dicarbonsäure-3-hexyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(2-n-butoxyethyl)-5-[2-(4,4-diphenylpiperidyl-1)-ethyl]-ester,
1,4-Dihydro-2,6-dimethyl-4-(3-nitrophenyl)-pyridin-3,5-dicarbonsäure-3-methyl-5-{2-[4,4-di (4-methoxyphenyl)-piperidyl-1]-ethyl}-ester,
1,4-Dihydro-2,6-dimethyl-4-(2-trifluormethylphenyl)-pyridin-3,5-dicarbonsäure-3-ethyl-5-[2-(4,4-diphenylpiperidyl-1)-ethyl]-ester,
1,4-Dihydro-2,6-dimethyl-4-[-3-(1,1,2,2-tetrafluorethoxy)-phenyl]-pyridin-3,5-dicarbonsäure-3-methyl-5-[2-(4,4-diphenylpiperidyl-1)-ethyl]-ester,
1,4-Dihydro-2,6-dimethyl-4-(2-difluormethoxyphenyl)-pyridin-3,5-dicarbonsäure-3-ethyl-5-[2-(4,4-diphenylpiperidyl-1)-ethyl]-ester,
1,4-Dihydro-2,6-dimethyl-4-(2-difluormethoxyphenyl)-pyridin-3,5-dicarbonsäure-3-ethyl-5-[4-(4,4-diphenylpiperidyl-1)-butyl]-ester,
1,4-Dihydro-2,6-dimethyl-4-(3-nitrophenyl)-pyridin-3,5-dicarbonsäure-3-methyl-5-[2-(4,4-dihydroxyphenylpiperidyl-1)-ethyl]-ester,
4-(2,3-Dichlorphenyl)-1,4-dihydro-2,6-dimethylpyridin-3,5-dicarbonsäure-3-methyl-5-[2-(4,4-diphenylpiperidyl-1)-ethyl]-ester,
4-(2,1,3-Benzoxdiazol-4-yl)-1,4-dihydro-2,6-dimethylpyridin-3,5-dicarbonsäure-3-methyl-5-[2-(4,4-diphenylpiperidyl-1)-ethyl]-ester,
4-(3-Cyanphenyl)-1,4-dihydro-2,6-dimethylpyridin-3,5-dicarbonsäure-3-methyl-5-[2-(4,4-diphenylpiperidyl-1)-ethyl]-ester,
1,4-Dihydro-2,6-dimethyl-4-(2-methoxyphenyl)-pyridin-3,5-dicarbonsäure-3-methyl-5-[2-(4,4-diphenylpiperidyl-1)-ethyl]-ester,
1,4-Dihydro-2,6-dimethyl-4-(2-pyridyl)-pyridin-3,5-dicarbonsäure-3-methyl-5-[2-(4,4-diphenylpiperidyl-1)-ethyl]-ester,
1,4-Dihydro-2,6-dimethyl-4-(5-methyl-2-thienyl)-pyridin-3,5-dicarbonsäure-3-methyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-methyl-5-{2-[4-(4-chlorphenyl)-4-phenylpiperidyl-1]-ethyl}-ester,
1,4-Dihydro-2,6-dimethyl-4-(2-nitrophenyl)-pyridin-3,5-dicarbonsäure-3-methyl-5-[3-(4,4-diphenylpiperidyl-1)-propyl]-ester,
1,4-Dihydro-2,6-diethyl-4-(3-nitrophenyl)-pyridin-3,5-dicarbonsäure-3-methyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-(propyl-2)-5-[3-(4,4-diphenylpiperidyl-1)-propyl]-ester,
1,4-Dihydro-2,6-dimethyl-4-(3-nitrophenyl)-pyridin-3,5-dicarbonsäure-3-hexyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-(2-n-butoxyethyl)-5-[3-(4,4-diphenylpiperidyl-1)-propyl]-ester,
1,4-Dihydro-2,6-dimethyl-4-(3-nitrophenyl)-pyridin-3,5-dicarbonsäure-3-methyl-5-{3-[4,4-di(4-methoxyphenyl)-piperidyl-1]-propyl}-ester,
1,4-Dihydro-2,6-dimethyl-4-(2-trifluormethylphenyl)-pyridin-3,5-dicarbonsäure-3-ethyl-5-[3-(4,4-diphenylpiperidyl-1)-propyl]-ester,
1,4-Dihydro-2,6-dimethyl-4-[3-(1,1,2,2-tetrafluorethoxy)-phenyl]-pyridin-3,5-dicarbonsäure-3-methyl-5-[3-(4,4-diphenylpiperidyl-1)-propyl]-ester,
1,4-Dihydro-2,6-dimethyl-4-(2-difluormethoxyphenyl)-pyridin-3,5-dicarbonsäure-3-ethyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-methyl-5-[3-(4,4-dihydroxyphenylpiperidyl-1)-propyl]-ester,
4-(2,3-Dichlorphenyl)-1,4-dihydro-2,6-dimethylpyridin-3,5-dicarbonsäure-3-methyl-5-[3-(4,4-diphenylpiperidyl-1)-propyl]-ester,
4-(2,1,3-Benzoxdiazol-4-yl)-1,4-dihydro-2,6-dimethylpyridin-3,5-dicarbonsäure-3-methyl-5-[3-(4,4-diphenylpiperidyl-1)-propyl]-ester,
4-(3-Cyanphenyl)-1,4-dihydro-2,6-dimethylpyridin-3,5-dicarbonsäure-3-methyl-5-[3-(4,4-diphenylpiperidyl-1)-propyl]-ester,
1,4-Dihydro-2,6-dimethyl-4-(2-methoxyphenyl)-pyridin-3,5-dicarbonsäure-3-methyl-5-[3-(4,4-diphenylpiperidyl-1)-propyl]-ester,
1,4-Dihydro-2,6-dimethyl-4-(2-pyridyl)-pyridin-3,5-dicarbonsäure-3-methyl-5-[3-(4,4-diphenylpiperidyl-1)-propyl]-ester,
1,4-Dihydro-2,6-dimethyl-4-(5-methyl-2-thienyl)-pyridin-3,5-dicarbonsäure-3-methyl-5-[3-(4,4-diphenylpiperidyl-1)-propyl]-ester, und
1,4-Dihydro-2,6-dimethyl-4-(3-nitrophenyl)-pyridin-3,5-dicarbonsäure-3-methyl-5-{3-[4-(4-chlorphenyl)-4-phenylpiperidyl-1]-propyl}-ester; und ihre Salze.

Die Verbindungen der Formel I besitzen an der 4-Position im 1,4-Dihydropyridin ein Chiralitätszentrum. Die Erfindung umfaßt daher sowohl die Enantiomeren und bei Vorliegen eines weiteren Chiralitätszentrums die Diastereomeren, als auch deren Gemische und Racemate.

Ein weiterer Gegenstand der Erfindung ist ein Verfahren zur Herstellung der erfindungsgemäßen Verbindungen und ihrer Salze. Das Verfahren ist dadurch gekennzeichnet, daß man

a) Zimtsäurederivate der Formel II

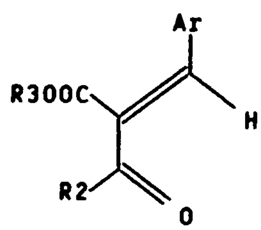

(II),

mit Enaminderivaten der Formel III

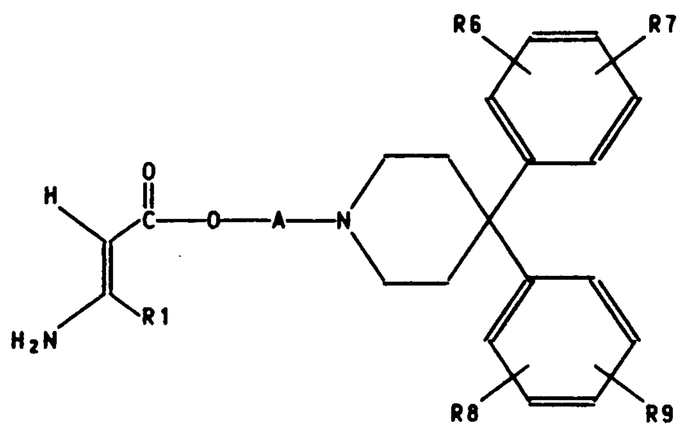

(III),

oder

b) Zimtsäurederivate der Formel II mit Ammoniak und $\beta$-Ketocarbonsäurederivaten der Formel IV

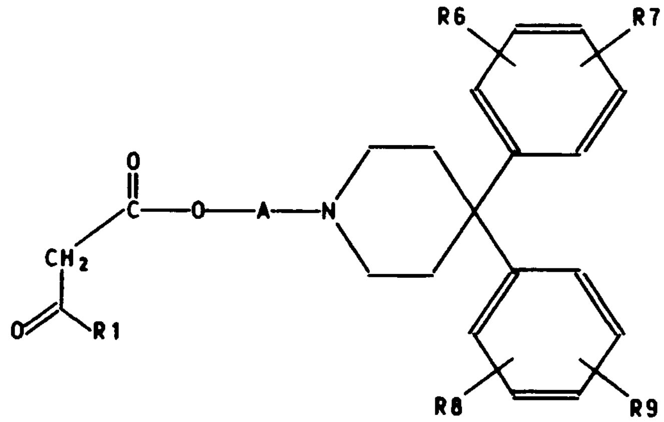

(IV),

oder

c) Enamine der Formel V

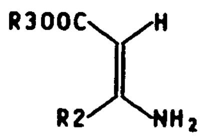

(V).

mit Benzylidencarbonsäurederivaten der Formel VI

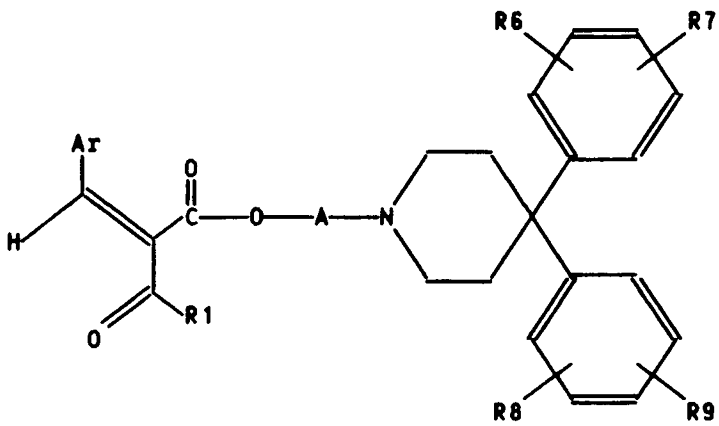

(VI),

oder

d) Ketoverbindungen der Formel VII

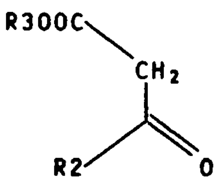

(VII),

mit Ammoniak und Benzylidencarbonsäurederivaten der Formel VI, oder
e) Aldehyde der Formel VIII

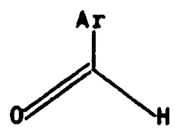

(VIII),

mit Enaminen der Formel V und $\beta$-ketocarbonsäurederivaten der Formel IV, oder
f) Aldehyde der Formel VIII mit Enaminderivaten der Formel III und Ketoverbindungen der Formel VII, oder
g) 1,4-Dihydropyridine der Formel IX

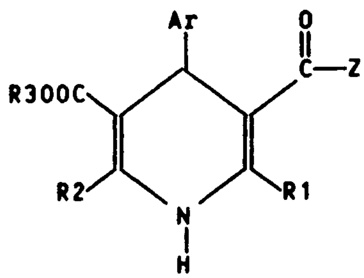

(IX),

mit Diarylverbindungen der Formel X

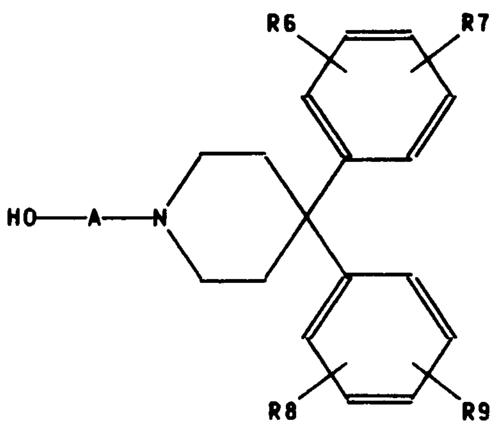

(X),

als solche(n) oder in Form ihrer Salze umsetzt und gewünschtenfalls anschließend erhaltene Salze in die freien Basen oder erhaltene Basen in die Salze überführt, wobei Ar, R1, R2, R3, R4, R5, R6, R7, R8, R9, Y und A die oben angegebenen Bedeutungen haben und Z gemeinsam mit der Carbonylgruppe, woran es gebunden ist, eine Carboxylgruppe oder ein reaktives Carbonsäurederivat (z. B. ein Carbonsäurehalogenid) darstellt.

Das Verfahren gemäß den Varianten a bis f wird in geeigneten, vorzugsweise inerten organischen Lösungsmitteln durchgeführt. Beispielsweise seien genannt Alkohole, wie Ethanol, Methanol, Isopropanol oder tert.-Butanol, Ether, wie Dioxan, Diethylether, Tetrahydrofuran, Glykolmonoethylether, Glykoldimethylether oder sonstige, beispielsweise polare Lösungsmittel wie Dimethylformamid, Dimethylsulfoxid, Acetonitril oder Hexamethylphosphorsäuretriamid, oder insbesondere chlorierte Kohlenwasserstoffe wie Methylenchlorid, Chloroform oder Tetrachlorethylen.

Die Reaktionstemperaturen können- je nach Reaktivität der Edukte- in einem weiten Bereich variieren. Im allgemeinen wird die Umsetzung bei Temperaturen zwischen 20 °C und 150 °C, vorzugsweise zwischen 20 °C und 100 °C, insbesondere bei der Siedetemperatur des verwendeten Lösungs mittels durchgeführt.

Das Verfahren kann bei Normaldruck oder bei erhöhtem Druck durchgeführt werden, wobei das Arbeiten bei Normaldruck die Regel ist und erhöhter Druck insbesondere bei Umsetzungen mit Ammoniak zur Anwendung kommen kann.

Bei der Durchführung des erfindungsgemäßen Verfahrens gemäß Varianten a bis f werden die an der Reaktion beteiligten Stoffe in der Regel jeweils in molaren Mengen eingesetzt, wobei jedoch - je nach Reaktionsbedingung - gewünschtenfalls auch ein Überschuß (beispielsweise an Ammoniak bei den Varianten b und d) eingesetzt werden kann.

Bei der Durchführung des Verfahrens gemäß Variante g kommen ähnliche Reaktionsbedingungen wie bei den Varianten a bis f zur Anwendung, jedoch sind - je nach Art des Substituenten Z gegebenenfalls zusätzliche Maßnahmen erforderlich. Stellt Z beispielsweise eine Hydroxylgruppe dar, so ist die Reaktion bevorzugt in Gegenwart eines wasserabspaltenden oder wasserbindenden Kondensationsmittels (wie z.B. Dicyclohexylcarbodiimid) durchzuführen. Stellt Z ein Halogenatom (z.B. ein Chloratom) dar, so ist die Reaktion gewünschtenfalls in Gegenwart einer Base (z. B. eines tertiären organischen Amins, wie Triethylamin, oder eines anorganischen Carbonates, wie Natriumcarbonat) durchzuführen.

Die Isolierung und Reinigung der erfindungsgemäßen Substanzen erfolgt in an sich bekannter Weise z. B. derart, daß man das Lösungsmittel im Vakuum abdestilliert und den erhaltenen Rückstand aus einem geeigneten Lösungsmittel umkristallisiert oder einer der üblichen Reinigungsmethoden, wie beispielsweise der Säulenchromatographie an geeignetem Trägermaterial, unterwirft.

Säureadditionssalze erhält man durch Auflösen der freien Base in einem geeigneten Lösungsmittel, z.B. in einem chlorierten Kohlenwasserstoff, wie Methylenchlorid oder Chloroform, oder einem niedermolekularen aliphatischen Alkohol (Ethanol, Isopropanol), das die gewünschte Säure enthält, oder dem die gewünschte Säure anschließend zugegeben wird.

Die Salze werden durch Filtrieren, Umfällen, Ausfällen mit einem Nichtlösungsmittel für das Anlagerungssalz oder durch Verdampfen des Lösungsmittels gewonnen.

Erhaltene Salze können durch Alkalisierung, z.B. mit wäßriger Ammoniaklösung, in die freien Basen umgewandelt werden, welche wiederum in Säureadditionssalze übergeführt werden können. Auf diese Weise lassen sich pharmakologisch nicht verträgliche Säureadditionssalze in pharmakologisch verträgliche Säureadditionssalze umwandeln.

Die Ausgangsverbindungen sind literaturbekannt oder können in Analogie zu literaturbekannten Methoden hergestellt werden. Die Zimtsäurederivate II und die Benzylidencarbonsäurederivate VI können beispielsweise in Analogie zu G. Jones ["The Knoevenagel Condensation" in Org. Reactions, Vol. XV, 204f (1967)] hergestellt werden. Die Enaminderivate III bzw. die Enamine V sind beispielsweise analog A.C. Cope [J. Amer. Chem. Soc. 67, 1017 (1945)] erhältlich. β-Ketocarbonsäurederivate IV und Ketoverbindungen VII können gemäß D. Borrmann ["Umsetzung von Diketen mit Alkoholen, Phenolen und Mercaptanen" in Houben-Weyl, Methoden der Organischen Chemie, Vol. VII/4, 230ff (1968)] oder Y. Oikawa et al. [J. Org. Chem. 43, 2087 (1978)] hergestellt werden. Die Verbindungen IX sind aus entsprechenden Ausgangsverbindungen analog Verfahrensvariante a bis f zugänglich. Verbindungen X sind durch Umsetzung entsprechender Piperidine (siehe z.B. DE-PS 19 36 452) mit ω-Halogenalkanolen erhältlich.

Die vorstehenden Herstellungsverfahren sind lediglich zur Verdeutlichung angegeben, und die Herstellung der erfindungsgemäßen Verbindungen der Formel I ist nicht auf diese Verfahren beschränkt. Vielmehr ist auch jede Modifikation dieser Verfahren in gleicher Weise für die Herstellung der erfindungsgemäßen Verbindungen anwendbar.

Bevorzugte Verfahrensvarianten sind die Varianten a und c.

Die folgenden Herstellungsbeispiele sollen die Erfindung näher erläutern, ohne sie einzuschränken. Schmp. bedeutet Schmelzpunkt, Kp. steht für Siedepunkt. Zers. bedeutet Zersetzung.

## Beispiele

1. 1,4-Dihydro-2,6-dimethyl-4-(3-nitrophenyl)-pyridin-3,5-dicarbonsäure-3-methyl-5-[3-(4,4-diphenylpiperidyl-1)-propyl]-ester-hydrochlorid

Verfahrensvariante e)

4,53 g 3-Nitrobenzaldehyd, 3,45 g 3-Aminocrotonsäuremethylester und 11,38 g Acetessigsäure-[3-(4,4-diphenylpiperidyl-1)-propyl]-ester in 100 ml 2-Propanol werden über Nacht unter Rückfluß zum Sieden erhitzt. Die abgekühlte Lösung wird zur Trockne eingeengt und der verbleibende Rückstand über eine Kieselgelsäule mit Essigsäureethylester als Eluens chromatographiert. Die einheitlichen Produktfraktionen hinterlassen nach dem Einengen einen festen aufgeschäumten Rückstand, der in Methanol gelöst und mit

etherischer Salzsäure versetzt wird. Die Lösung wird eingeengt, der verbleibende feste Rückstand in wenig Methanol aufgenommen und die Titelsubstanz durch Zugabe von Petrolether ausgefällt. Schmp.: ab 135 °C (Zersetzung); Ausbeute: 9,3 g.

Die Ausgangsverbindungen werden wie folgt erhalten:

a) Acetessigsäure-[3-(4,4-diphenylpiperidyl-1)-propyl]-ester

23,6 g 3-(4,4-Diphenylpiperidyl-1)-propanol werden in 100 ml abs. Toluol gelöst und unter Rühren mit 16 ml einer 50%igen Diketenlösung in Aceton versetzt. Nach mehrtägigem Stehen bei Raumtemperatur (DC-Kontrolle) wird der Ansatz eingeengt und der Rückstand im Hochvakuum getrocknet. Das verbleibende hellgelbe, zähe Öl wird ohne weitere Reinigung für die nächste Stufe eingesetzt.

b) 3-(4,4-Diphenylpiperidyl-1)-propanol

40 g 4,4-Diphenylpiperidin, 24,7 g 3-Brompropanol, 116,4 g pulverisiertes Kaliumcarbonat und ca. 1 g Kaliumiodid werden in 500 ml eines 1:1 Gemisches aus Dioxan und 1-Butanol ca. 48 Stunden unter Rückfluß und kräftigem Rühren zum Sieden erhitzt. Nach dem Abkühlen wird filtriert, das Filtrat eingeengt. Der ölige Rückstand wird in Essigsäureethylester aufgenommen und die Lösung nochmals filtriert. Nach dem Einengen des Filtrats zur Trockne erhält man das Produkt als gelblichen öligen Rückstand, der langsam wachsartig fest wird. Ausbeute: 44,8 g. Mit etherischer Salzsäure erhält man das Hydrochlorid, das in 2-Propanol umkristallisiert wird. Schmp.: 226-7 °C.

Alternativ wird die Ausgangsverbindung b) erhalten, wenn 352 g 4,4-Diphenylpiperidin, 128 g Natriumhydroxidgranulat, 2,5 l Methylenchlorid, 500 ml Wasser, 218 g 3-Brom-1-propanol und katalytische Mengen eines Phasentransferkatalysators (z.B. Benzyltrimethylammoniumchlorid) 10 Stunden unter Rückfluß zum Sieden erhitzt werden. Die abgetrennte organische Phase wird mit Wasser gewaschen, die gesammelten Wasserphasen werden mit Methylenchlorid extrahiert. Nach dem Trocknen der vereinigten organischen Phasen mit Natriumsulfat wird die klare, bräunliche Lösung zur Trockne eingedampft. Der harzige braune Rückstand wird in 4,5 l siedendem Petrolether (Siedebereich 100-140 °C) aufgenommen, die Lösung heiß von unlöslichem Rückstand abfiltriert und abgekühlt. Nach Stehenlassen über Nacht erhält man die Titelverbindung als freie Base in farblosen, groben Kristallen. Schmp.: 97 °C. Ausbeute: 303 g.

Analog wurden folgende Ausgangsverbindungen hergestellt:
4-(4,4-Diphenylpiperidyl-1)-butanol, Schmp. des Hydrochlorids 209-212 °C,
2-(4,4-Diphenylpiperidyl-1)-2-methylpropanol, Schmp. 115-116 °C,
3-[4,4-Di-(4-methoxiphenyl)-piperidyl-1]-propanol, Schmp. des Hydrochlorids 130-134 °C (enthält 1 Äquivalent Methanol im Kristall),
3-(4,4-Diphenylpiperidyl-1)-2-methyl-2-propanol, Schmp. des Hydrochlorids 181-183 °C,
2-(4,4-Diphenylpiperidyl-1)-ethanol, Schmp. des Hydrochlorids 197-199 °C.

Durch Umsetzung der vorstehend genannten Alkohole mit Diketen-Lösung analog Beispiel 1a erhält man die entsprechenden Acetesigsäureesterderivate, die ohne weitere Reinigung für die nächste Stufe eingesetzt werden.

Verfahrensvariante c)

15,38 g 2-Acetyl-3-(3-nitrophenyl)-acrylsäure-[3-(4,4-diphenylpiperidyl-1)-propyl]-ester und 3,45 g 3-Aminocrotonsäuremethylester in 100 ml 2-Propanol werden über Nacht unter Rückfluß zum Sieden erhitzt. Die abgekühlte Lösung wird zur Trockne eingeengt, der aufgeschäumte feste Rückstand in wenig Methylenchlorid aufgenommen und mit etherischer Salzsäure versetzt. Nach erneutem Einengen zur Trockne und Aufnehmen des festen Rückstandes in wenig Methylenchlorid setzt man soviel Essigsäureethylester zu, bis eine leichte Trübung bestehen bleibt. Nach Stehen im Kühlschrank kristallisiert die Titelverbindung über Nacht in feinen, leicht gelblich gefärbten Schuppen (Mikroskop) aus. Schmp.: 230-231 °C (Zers.); Ausbeute: 13,6 g.

Als weitere Lösungsmittel für die Kondensationsreaktion werden eingesetzt: tert. -Butanol, Dioxan, Tetrahydrofuran, chlorierte Kohlenwasserstoffe. Die Ausbeuten an Produkt betragen 60-80 % der Theorie.

Als weitere Salze der Titelverbindung 1 werden hergestellt:
Hydrobromid: Schmp.: 229-230 °C (Zers.), feine Plättchen (aus Essigsäureethylester und Diisopropylether);
Fumarat: Schmp.: 144-145 °C (Zers.), feine Schuppen (aus Essigsäureethylester);
Maleat: Schmp.: 151-152 °C (Zers.), grobe Nadelbüschel (aus Essigsäureethylester).

Die freie Base der Titelverbindung 1 erhält man, wenn der Kondensationsansatz zur Trockne eingeengt und der aufgeschäumte feste Rückstand in wenig Methylenchlorid aufgenommen wird; nach Zusatz von Diisopropylether bis zur bleibenden feinen Trübung kristallisiert die Base nach Stehen im Kühlschrank in feinen Plättchen aus. Schmp.: 145-147 °C.

Die Ausgangsverbindung 2-Acetyl-3-(3-nitrophenyl)-acrylsäure-[3-(4,4-diphenylpiperidyl-1)-propyl]-ester (cis-/trans-Isomerengemisch) wird wie folgt erhalten:
40,14 g Acetessigsäure-[3-(4,4-diphenylpiperidyl-1)-propyl]-ester, 15,97 g 3-Nitrobenzaldehyd, 8,0 ml Essigsäure und 0,5 ml Piperidin werden in 300 ml Benzol am Wasserabscheider zum Sieden erhitzt. Nach Abscheiden von 1,9 ml Wasser wird die abgekühlte Lösung mit gesättigter Natriumhydrogencarbonatlösung und anschließend mit Wasser gewaschen. Nach dem Trocknen der organischen Phase mit Natriumsulfat engt man die klare rot-braune Lösung im Hochvakuum bis zur Gewichtskonstanz ein. Der erhaltene rotbraune zäh-viskose Rückstand wird direkt ohne weitere Reinigung zur Kondensation eingesetzt. Ausbeute: 52 g Rohprodukt. Als Schlepper sind ferner geeignet: Toluol, chlorierte Kohlenwasserstoffe. Die Ausbeute an Rohprodukt beträgt 90-100 % der Theorie.

Durch Umsetzung der freien Base mit equimolarer Menge Fumarsäure erhält man das Fumarat der Ausgangsverbindung; Schmp.: ab 128 °C (Zers.), feine Nadelbüschel, aus Essigsäureethylester.

Durch Umsetzung mit etherischer Salzsäure wird das Hydrochlorid erhalten: Schmp.: 152-155 °C (feine Plättchen, aus Essigsäureethylester und Diethylether).

Verfahrensvariante a

134,6 g 2-Acetyl-3-(3-nitrophenyl)-acrylsäuremethylester, 204,5 g 3-Aminocrotonsäure-[3-(4,4-diphenylpiperidyl-1)-propyl]-ester und 4,5 ml Essigsäure in 2,7 l wasserfreiem Dioxan werden unter einer Stickstoffathmosphäre 20 Stunden unter Rückfluß zum Sieden erhitzt. Nach Abkühlen wird die Mischung mit 45 ml konzentrierter Salzsäure (37 %) versetzt, Impfkristalle der Titelverbindung 1 zugegeben und 24 Stunden bei Raumtemperatur stehengelassen. Es wird abgesaugt, mit Dioxan und Diisopropylether gewaschen und dann im Vakuum bei 75 °C getrocknet. Das Produkt (270 g) wird zwischen 1,5 l Methylenchlorid und wässeriger Ammoniaklösung (pH 11) verteilt, die organische Phase über Natriumsulfat getrocknet und dann das Lösungsmittel im Vakuum abdestilliert. Der Rückstand wird in 2,5 l Dioxan gelöst, dann wird mit 35 ml konzentrierter Salzsäure (37%) versetzt, angeimpft und 40 Stunden stehengelassen. Das auskristallisierte Produkt wird abgesaugt, mit Dioxan und anschließend Diisopropylether gewaschen und bei 100 °C im Vakuum getrocknet. Man erhält die Titelsubstanz als hellgelbes Pulver (Mikroskop: Kleine Nadeln).
Schm.: 198-200 °C; Ausbeute: 246 g.

Die Ausgangsverbindung 3-Aminocrotonsäure-[3-(4,4-diphenylpiperidyl-1)-propyl]-ester wird wie folgt hergestellt:
260,5 g Acetessigsäure-[3-(4,4-diphenylpiperidyl-1)-propyl]-ester in 1,6 l 2-Propanol werden über Nacht mit 260 ml konzentrierter Ammoniaklösung gerührt. Der ausgefallene feine, leicht ockerfarbige Niederschlag wird abgesaugt und mit kaltem 2-Propanol, Diethylether und schließlich Petrolether gewaschen. Schmp.: 144-150 °C; Ausbeute: 225 g. Nach Zusatz von weiteren 100 ml konzentrierter Ammoniaklösung zum Filtrat erhält man nach mehrtägigem Stehen im Kühlschrank weitere 12 g Produkt mit identischem Schmelzpunkt.

Alternativ wird die Ausgangsverbindung erhalten, wenn zur Lösung von Acetessigsäure-[3-(4,4-diphenylpiperidyl-1)-propyl]-ester in 2-Propanol unter Rühren solange gasförmiges Ammoniak eingeleitet wird, bis kein weiterer Niederschlag mehr ausfällt. Ausbeute ca. 90 % der Theorie.

2. 1,4-Dihydro-2,6-dimethyl-4-(3-nitrophenyl)-pyridin-3,5-dicarbonsäure-3-methyl-5-[2-(4,4-diphenylpiperidyl-1)-ethyl]-ester-hydrochlorid

Analog Beispiel 1 erhält man aus 4,53 g 3-Nitrobenzaldehyd, 3,45 g Aminocrotonsäuremethylester und 11 g Acetessigsäure-[2-(4,4-diphenylpiperidyl-1)-ethyl]-ester in 100 ml 2-Propanol die Titelverbindung vom Schmp.: ab 137 °C (Zersetung); Ausbeute: 8,5 g.

3. 4-(3-Cyanphenyl)-1,4-dihydro-2,6-dimethylpyridin-3,5-dicarbonsäure-3-methyl-5-[3-(4,4-diphenylpiperidyl-1)-propyl]-ester-hydrochlorid

Analog Beispiel 1 erhält man aus 3,93 g 3-Cyanbenzaldehyd, 3,45 g 3-Aminocrotonsäuremethylester und 11,38 g Acetessigsäure-[3-(4,4-diphenylpiperidyl-1)-propyl]-ester in 80 ml tert.-Butanol die Titelverbindung vom Schmp. 136-146 °C (langsames Zerfließen, amorph, in Petrolether ausgefällt); Ausbeute: 5,9 g.

4. 1,4-Dihydro-2,6-dimethyl-4-(3-nitrophenyl)-pyridin-3,5-dicarbonsäure-3-methyl-5-[2-(4,4-diphenylpiperidyl-1)-2-methylpropyl]-ester-hydrochlorid

Analog Beispiel 1 erhält man aus 15,8 g 2-Acetyl-3-(3-nitrophenyl)-acrylsäure-[2-(4,4-diphenylpiperidyl-1)-2-methylpropyl]-ester und 3,45 g 3-Aminocrotonsäuremethylester in 100 ml Tetrahydrofuran nach 12 Stunden Reaktionsdauer die Titelverbindung vom Schmp. ab 155 °C (langsames Zerfließen, amorph, in Petrolether ausgefällt); Ausbeute: 13,6g.

5. 1,4-Dihydro-2,6-dimethyl-4-(3-nitrophenyl)-pyridin-3,5-dicarbonsäure-3-ethyl-5-[3-(4,4-diphenylpiperidyl-1)-propyl]-ester-hydrochlorid

Analog Beispiel 1 erhält man aus 13,16 g 2-Acetyl-3-(3-nitrophenyl)-acrylsäureethylester und 18,91 g 3-Aminocrotonsäure-[3-(4,4-diphenylpiperidyl-1)-propyl]-ester in 100 ml Tetrahydrofuran nach 6 Stunden Reaktionsdauer die Titelverbindung vom Schmp. 230-232 °C (feine eckige Kristalle, aus Acetonitril und Diethylether); Ausbeute: 29,7 g.

6. 1,4-Dihydro-2,6-dimethyl-4-[3-(1,1,2,2-tetrafluorethoxi)-phenyl]-pyridin-3,5-dicarbonsäure-3-methyl-5-[3-(4,4-diphenylpiperidyl-1)-propyl]-ester-hydrochlorid

Analog Beispiel 1 erhält man aus 40,33 g 2-Acetyl-3-[3-(1,1,2,2-tetrafluorethoxi)-phenyl]-acrylsäuremethylester und 37,85 g 3-Aminocrotonsäure-[3-(4,4-diphenylpiperidyl-1)-propyl]-ester in 400 ml Tetrahydrofuran und 0,5 ml Eisessig nach 14 Stunden Reaktionsdauer die Titelverbindung vom Schmp. 189-192 °C; Ausbeute: 57 g.

7. 1,4-Dihydro-2,6-dimethyl-4-[3-(1,1,2,2-tetrafluorethoxi)-phenyl]-pyridin-3,5-dicarbonsäure-3-methyl-5-[2-(4,4-diphenylpiperidyl-1)-ethyl]-ester-hydrochlorid

Analog Beispiel 1 erhält man aus 4,03 g 2-Acetyl-3-[3-(1,1,2,2-tetrafluorethoxi)-phenyl]-acrylsäuremethylester und 3,4 g 3-Aminocrotonsäure-[2-(4,4-diphenylpiperidyl-1)-ethyl]-ester in 80 ml 2-Propanol nach 8 Stunden Reaktionsdauer die Titelverbindung vom Schmp. 130-140 °C (langsames Zerfließen, amorph, ausgefällt in Petrolether und Diethylether 1:1); Ausbeute: 6,2 g.

8. 1,4-Dihydro-2,6-dimethyl-4-(3-nitrophenyl)-pyridin-3,5-dicarbonsäure-3-(2-methoxiethyl)-5-[2-(4,4-diphenylpiperidyl-1)-ethyl]-ester-fumarat

Analog Beispiel 1 erhält man aus 4,99 g 2-Acetyl-3-(3-nitrophenyl)-acrylsäure-[2-(4,4-diphenylpiperidyl-1)-ethyl]-ester und 1,6 g 3-Aminocrotonsäure-2-(2-methoxiethyl)-ester in 60 ml Tetrahydrofuran und 0,5 ml Eisessig nach 4 Stunden Reaktionsdauer die Titelverbindung vom Schmp. ab 130 °C (langsames Zerfließen, feine Plättchen, aus Essigsäureethylester und Diethylether); Ausbeute: 3,7 g.

9. 1,4-Dihydro-2,6-dimethyl-4-(3-nitrophenyl)-pyridin-3,5-dicarbonsäure-3-(2-methoxiethyl)-5-[3-(4,4-diphenylpiperidyl-1)-propyl]-ester-fumarat

Analog Beispiel 1 erhält man aus 5,12 g 2-Acetyl-3-(3-nitrophenyl)-acrylsäure-[3-(4,4-diphenylpiperidyl-1)-propyl]-ester und 1,6 g 3-Aminocrotonsäure-2-(2-methoxiethyl)-ester in 80 ml tert.-Butanol nach 5 Stunden Reaktionsdauer die Titelverbindung vom Schmp. 184-185 °C (kantige Schuppen, aus Essigsäureethylester und Diethylether); Ausbeute: 5,3 g.

10. 1,4-Dihydro-2,6-dimethyl-4-(3-nitrophenyl)-pyridin-3,5-dicarbonsäure-3-methyl-5-{3-[4,4-di-(4-methoxiphenyl)-piperidyl-1]-propyl}-ester-hydrochlorid

Analog Beispiel 1 erhält man aus 4,4 g Acetessigsäure-3-[4,4-di-(4-methoxiphenyl)-piperidyl-1]-propylester, 1,15 g 3-Aminocrotonsäuremethyl-ester und 1,51 g 3-Nitrobenzaldehyd in 60 ml tert.-Butanol nach 12 Stunden Reaktionsdauer die Titelverbindung vom Schmp. ab 138 °C (langsames Zerfließen, amorph, ausefällt in Petrolether); Ausbeute: 4,9 g.

11. 1,4-Dihydro-2,6-dimethyl-4-(3-nitrophenyl)-pyridin-3,5-dicarbonsäure-3-methyl-5-[4-(4,4-diphenylpiperidyl-1)-butyl]-ester-fumarat

Analog Beispiel 1 erhält man aus 3,94 g Acetessigsäure -[4-(4,4-diphenylpiperidyl-1)-butyl]-ester, 1,15 g 3-Aminocrotonsäuremethylester und 1,51 g 3-Nitrobenzaldehyd in 80 ml tert.-Butanol nach 6 Stunden Reaktionsdauer die Titelverbindung vom Schmp. 123-126° (feine Nadeln, aus Essigsäureethylester und Methylenchlorid); Ausbeute: 3,9 g.

12. 1,4-Dihydro-2,6-dimethyl-4-(3-nitrophenyl)-pyridin-3,5-dicarbonsäure-3-methyl-5-[1,1-dimethyl-2-(4,4-diphenylpiperidyl-1)-ethyl]-ester-hydrochlorid

Analog Beispiel 1 erhält man aus 15,8 g 2-Acetyl-3-(3-nitrophenyl)-acrylsäure-[1,1-dimethyl-2-(4,4-diphenylpiperidyl-1)-ethyl]-ester und 3,45 g 3-Aminocrotonsäuremethylester in 120 ml 2-Propanol nach 15 Stunden Reaktionsdauer die Titelverbindung vom Schmp. ab 148°C (langsames Zerfließen, amorph, ausgefällt in Petrolether); Ausbeute: 11,3 g.

13. 1,4-Dihydro-2,6-dimethyl-4-(2-difluormethoxiphenyl)-pyridin-3,5-dicarbonsäure-3-ethyl-5-[3-(4,4-diphenylpiperidyl-1)-propyl]-ester-hydrochlorid

Analog Beispiel 1 erhält man aus 3,8 g 2-Acetyl-3-(2-difluormethoxiphenyl)-acrylsäure-[3-(4,4-diphenylpiperidyl-1)-propyl]-ester und 1,3 g 3-Aminocrotonsäureethylester in 60 ml tert.-Butanol nach 20 Stunden Reaktionsdauer die Titelverbindung vom Schmp. ab 126°C (langsames Zerfließen, amorph, ausgefällt in Petrolether und Diethylether 1:1); Ausbeute: 2,8 g.

14. 1,4-Dihydro-2,6-dimethyl-4-(3-nitrophenyl)-pyridin-3,5-dicarbonsäure-3-[3-(4,4-diphenylpiperidyl-1)-propyl]-ester

6,8 g 1,4-Dihydro-2,6-dimethyl-4-(3-nitrophenyl)-pyridin-3,5-dicarbonsäure-3-(2-cyanethyl)-5-[3-(4,4-diphenylpiperidyl-1)-propyl]-ester-hydrochlorid werden mit 40 ml 0,5 N Natronlauge und 100 ml Dioxan 3 Stunden bei Raumtemperatur gerührt. Nach Abdestillieren der Hauptmenge Dioxan werden 15 ml 2 N Salzsäure zum Rückstand zugesetzt. Die milchig-trübe Lösung wird 4 mal mit je 100 ml Chloroform/n-Butanol (3:1) extrahiert und die vereinigte organische Phase mit 50 ml gesättigter Kochsalzlösung gewaschen. Die organische Phase wird zur Trockne eingeengt und der feste Rückstand in Chloroform/Methanol (1:1) umkristallisiert. Schmp. 192-195°C (Zersetzung); Ausbeute: 5,4 g.

Die Ausgangsverbindung 1,4-Dihydro-2,6-dimethyl-4-(3-nitrophenyl)-pyridin-3,5-dicarbonsäure-3-(2-cyanethyl)-5-[3-(4,4-diphenylpiperidyl-1)-propyl]-ester wird wie folgt erhalten:

8,1 g 2-Acetyl-3-(3-nitrophenyl)-acrylsäure-2-cyanethylester und 10,6 g 3-Aminocrotonsäure-[3-(4,4-diphenylpiperidyl-1)-propyl]-ester werden in 120 ml 2-Propanol und 0,5 ml Eisessig 4 Stunden unter Rückfluß zum Sieden erhitzt. Nach dem Abdestillieren der Hauptmenge Lösungsmittel wird der Rückstand nach Zugabe von je 50 ml Toluol 2 mal zur Trockne eingeengt. Der fest aufgeschäumte Rückstand wird in Isopropanol aufgenommen und die klare Lösung bis zur ersten bleibenden Trübung mit Diethylether versetzt. Nach Stehenlassen im Kühlschrank kristallisiert die Ausgangsverbindung in feinen Plättchen aus. Schmp.: 158-160°C; Ausbeute: 14,3 g.

Mit etherischer Salzsäure erhält man das Hydrochlorid der Ausgangsverbindung, das in Methylenchlorid/Methanol umkristallisiert wird. Schmp.: 184-194°C (langsames Zerfließen).

15. 1,4-Dihydro-2,6-dimethyl-4-[3-(1,1,2,2-tetrafluorethoxi)-phenyl]-pyridin-3,5-dicarbonsäure-3-ethyl-5-[3-(4,4-diphenylpiperidyl-1)-propyl]-ester-hydrochlorid

Analog Beispiel 1 erhält man aus 22,2 g 3-(1,1,2,2-Tetrafluorethoxi)-benzaldehyd, 12,9 g 3-Aminocrotonsäureethylester und 37,9 g Acetessigsäure-[3-(4,4-diphenylpiperidyl-1)-propyl]-ester in 280 ml tert.-Butanol nach 14 Stunden Reaktionsdauer die Titelverbindung vom Schmp. 196-197°C (Zersetzung, aus Methylenchlorid und Diisopropylether); Ausbeute: 48,9 g.

16. 1,4-Dihydro-2,6-dimethyl-4-(3-difluormethoxiphenyl)-pyridin,-3,5-dicarbonsäure-3-ethyl-5-[3-(4,4-diphenylpiperidyl-1)-propyl]-ester-hydrochlorid

Analog Beispiel 1 erhält man aus 1,6 g 3-Difluormethoxibenzaldehyd, 1,3 g 3-Aminocrotonsäureethylester und 3,8 g Acetessigsäure-[3-(4,4-diphenylpiperidyl-1)-propyl]-ester in 80 ml Tetrahydrofuran nach 4 Stunden Reaktionsdauer die Titelverbindung vom Schmp. 197-198 °C (feine Plättchen aus Methylenchlorid und Essigsäureethylester); Ausbeute: 5,1 g.

17.4-(2,3-Dichlorphenyl)-1,4-dihydro-2,6-dimethylpyridin-3,5-dicarbonsäure-3-methyl-5-[3-(4,4-diphenylpiperidyl-1)-propyl]-ester-hydrochlorid

Analog Beispiel 1 erhält man aus 2,72 g 2-Acetyl-3-(2,3-dichlorphenyl)-acrylsäuremethylester und 3,79 g 3-Aminocrotonsäure-[3-(4,4-diphenylpiperidyl-1)-propyl]-ester in 100 ml 2-Propanol nach 7 Stunden Reaktionszeit die Titelverbindung vom Schmp. 228-230 °C (feine Nadeln aus Methanol und Essigsäureethylester); Ausbeute: 4,2 g.

18. 4-(2,1,3-Benzoxdiazol-4-yl)-1,4-dihydro-2,6-dimethyl-pyridin-3,5-dicarbonsäure-3-methyl-5-[3-(4,4-diphenylpiperidyl-1)-propyl]-ester-hydrochlorid

Analog Beispiel 1 erhält man aus 2,5 g 2-Acetyl-3-(2,1,3-benzoxdiazol-4-yl)-acrylsäuremethylester und 3,8 g 3-Aminocrotonsäure-[3-(4,4-diphenylpiperidyl-1)-propyl]-ester in 60 ml Tetrahydrofuran und 0,5 ml Essigsäure nach 4 Stunden Reaktionsdauer die Titelverbindung vom Schmp. 208-210 °C (feine, klumpige Kristalle, aus Essigsäureethylester/Methylenchlorid);Ausbeute 4,1 g.

19. 1,4-Dihydro-2,6-dimethyl-4-(3-fluorphenyl)-pyridin-3,5-dicarbonsäure-3-methyl-5-[3-(4,4-diphenylpiperidyl-1)-propyl]-ester-hydrochlorid

Analog Beispiel 1 erhält man aus 2,44 g 2-Acetyl-3-(3-fluorphenyl)-acrylsäuremethylester und 4,39 g 3-Aminocrotonsäure-[3-(4,4-diphenylpiperidyl-1)-propyl]-ester in 50 ml Tetrahydrofuran nach 5 Stunden Reaktionsdauer die Titelverbindung vom Schmp. 213-216 °C (feine, kantige Kristalle, aus Methylenchlorid/Diisopropylether); Ausbeute 4,8 g.

20. 1,4-Dihydro-2,6-dimethyl-4-(2-trifluromethylphenyl)-pyridin-3,5-dicarbonsäure-3-methyl-5-[3-(4,4-diphenylpiperidyl-1)-propyl]-ester-hydrochlorid

Analog Beispiel 1 erhält man aus 2,7 g 2-Acetyl-3-(2-trifluormethylphenyl)-acrylsäuremethylester und 4,4 g 3-Aminocrotonsäure-[3-(4,4-diphenylpiperidyl-1)-propyl]-ester in 60 ml tert.-Butanol nach 4 Stunden Reaktionsdauer die Titelverbindung vom Schmp. 158-162 °C (feine, würfelige Kristalle, aus Methylenchlorid/Diisopropylether); Ausbeute 4,2 g.

21. 4-(2-Cyanphenyl)-1,4-dihydro-2,6-dimethylpyridin-3,5-dicarbonsäure-3-ethyl-5-[3-(4,4-diphenylpiperidyl-1)-propyl]-ester-hydrochlorid

Analog Beispiel 1 erhält man aus 3,93 g 2-Cyanbenzaldehyd, 3,45g 3-Aminocrotonsäuremethylester und 11,38 g Acetessigsäure-[3-(4,4-diphenylpiperidyl-1)-propyl]-ester in 80 ml 2-Propanol nach 10 Stunden Reaktionsdauer die Titelverbindung vom Schmp. 178-181 °C (feine Nadeln, aus Essigsäureethylester/Methylenchlorid); Ausbeute 8,4 g.

22. 4-(2-Chlorphenyl)-1,4-dihydro-2,6-dimethylpyridin-3,5-dicarbonsäure-3-methyl-5-[3-(4,4-diphenylpiperidyl-1)-propyl]-ester-hydrochlorid

Analog Beispiel 1 erhält man aus 3,6 g 2-Acetyl-3-(2-chlorphenyl)-acrylsäuremethylester, 5,7 g 3-Aminocrotonsäure-[3-(4,4-diphenylpiperidyl-1)-propyl]-ester in 20 ml Tetrahydrofuran und 0,9 ml Essigsäure nach 8 Stunden Reaktionsdauer die Titelverbindung vom Schmp. 150 °C (Zers.) (feine Nadeln, aus Methylenchlorid/Diisopropylether); Ausbeute 3,3 g.

Gewerbliche Anwendbarkeit

Die erfindungsgemäßen Verbindungen der Formel I und ihre Salze besitzen wertvolle Eigenschaften, die sie gewerblich verwertbar machen. Sie stellen insbesondere wirksame Vasodilatoren mit coronartherapeutischen Eigenschaften dar. Die pharmakologische Wirksamkeit der erfindungsgemäßen Verbindungen, die gepaart ist mit einer geringen Toxizität, zeigt sich insbesondere in einer langsam eintretenden, starken und langanhaltenden Blutdrucksenkung. Darüberhinaus besitzen die erfindungsgemäßen Verbindungen peripher, coronar, cerebral und renal gefäßerweiternde sowie salidiuretische Eigenschaften.

In ihrer ausgezeichneten Wirksamkeit, die gepaart ist mit einer geringen Toxizität und dem Fehlen wesentlicher Nebenwirkungen, unterscheiden sich die erfindungsgemäßen Verbindungen in überraschender und vorteilhafter Weise von den Verbindungen des Standes der Technik. Als vorteilhafte Eigenschaften sind beispielsweise zu nennen: der langsame Eintritt der Blutdrucksenkung, das Ausmaß der Blutdrucksenkung, das lange Anhalten der Blutdrucksenkung, die gute Steuerbarkeit der Blutdrucksenkung, die nur geringe und bei wiederholter Gabe verschwindende Herzfrequenzsteigerung, die ausgezeichnete Bioverfügbarkeit, die große therapeutische Breite, das Fehlen zentraler Nebenwirkungen, das Fehlen kinetischer Interaktionen mit anderen Substanzen, das Ausbleiben einer Toleranzentwicklung, die ausgewogenen physikalischen Eigenschaften und die große Stabilität.

Die ausgezeichnete Wirksamkeit der erfindungsgemäßen Verbindungen der Formel I und ihrer Salze gestattet ihren Einsatz in der Humanmedizin, wobei als Indikation insbesondere primäre (essentielle) und sekundäre Hypertonien aller Schweregrade, koronare Herzkrankheiten (Koronarinsuffizienz, Angina Pectoris, Myocardinfarkt etc.), periphere und cerebrale Zirkulationsstörungen (Gehirnschlag, temporäre cerebrale Durchblutungsstörungen, renale Arterienverengung etc.), Herzinsuffiziens und Krankheiten, die auf einer erhöhten Wasser- und Natriumretention beruhen, in Betracht kommen.

Gegenstand der Erfindung sind außerdem die Verbindungen der Formel I zur Anwendung bei der Behandlung der genannten Krankheiten.

Ebenso umfaßt die Erfindung die Verwendung von Verbindungen der Formel I bei der Herstellung von Arzneimitteln, die zur Bekämpfung der genannten Krankheiten eingesetzt werden.

Ein weiterer Gegenstand der Erfindung sind Arzneimittel, die eine oder mehrere Verbindungen der allgemeinen Formel I enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (=Wirkstoffe) entweder als solche, oder vorzugsweise in Kombination mit geeigneten pharmazeutischen Hilfsstoffen in Form von Tabletten, Dragees, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95% beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral oder parenteral (insbesondere perlingual, intravenös oder percutan) appliziert werden.

Im allgemeinen hat es sich in der Humanmedizin als vorteilhaft erwiesen, den oder die Wirkstoffe bei oraler Gabe in einer Tagesdosis von etwa 0,01 bis etwa 10, vorzugsweise 0,05 bis 5 mg/kg Körpergewicht, gewünschtenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Bei einschleichender Dosierung wird zu Beginn der Behandlung eine geringere Dosis verabreicht, dann langsam auf eine höhere Dosis übergegangen. Nach Erreichen des gewünschten Therapieerfolges wird wieder auf eine niedrigere Dosis zurückgegangen.

Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der genannten Krankheiten eingesetzt werden, so können die pharmazeutischen Zubereitungen auch einen oder mehrere andere pharmakologisch aktive Bestandteile anderer Arzneimittelgruppen, wie andere Vasodilatoren, Antihypertensiva, $\alpha$-Rezeptorenblocker, $\beta$-Rezeptorenblocker, ACE-Hemmstoffe, Nitroverbindungen, Cardiotonika, Diuretika, Saluretika, Alkaloide, etc., wie Nifedipin, Dihydralazin, Prazosin, Propranolol, Labetalol, Captopril, Insosorbiddinitrat, Digoxin, Mefrusid, Clopamid, Spironolacton, Chlorthalidon, Furosemid, Polythiazid, Hydrochlorothiazid, Reserpin, Dihydroergocristin, Rescinnamin, Rauwolfia-Gesamtalkaloide etc. enthalten.

Pharmakologie

Die antihypertensive Wirksamkeit der erfindungsgemäßen Verbindungen kann am Modell der spontan hypertonen Ratte nachgewiesen werden.

Zur Bestimmung der antihypertensiven Wirkung werden die unten aufgeführten Verbindungen in den angegebenen Dosen an vier aufeinander folgenden Tagen an je 6 Ratten (Stamm SHR/N/Ibm/Bm ♂, 250-350 g) mit genetisch bedingtem Hochdruck (RR > 180 mmHg) täglich einmal mittels Schlundsonde verabfolgt. Die Messung des Blutdrucks erfolgt jeweils 6 und gegebenenfalls 2 oder 24 Stunden nach Substanzgabe.

Die Blutdruckmessung wird in einer Wärmekammer bei 36°C vorgenommen, um eine bessere Durchblutung der Schwanzarterie zu erreichen. Hierzu werden die Tiere in perforierte Lochblechkäfige verbracht und 20 - 40 Min. nach Beginn der Aufwärmung gemessen. Zur Messung des systolischen arteriellen Drucks wird eine ringförmige Manschette mit aufblasbarer Gummimembran zur Unterbindung der Durchblutung und ein ringförmiger Piezokristallaufnehmer zur Erfassung der Pulswellen auf den Schwanz aufgeschoben. Nach erfolgter Unterbindung des Blutstroms in der Schwanzarterie wird der Manschettendruck kontinuierlich reduziert. Die Wiederkehr der Pulswellen bei Druckablassen wird automatisch als systolischer Blutdruck erkannt und ausgedruckt (Bühler, R. et al.: Microprocessor-based automation of blood pressure measurement in the conscious rat. Proceedings of the 4th international symposium on rats with spontaneous hypertension and related studies, Rascher, R. et al. (Eds.), Schattauer Verlag, Stuttgart, New York, 1982, S. 410-413). Pulssignale und Druckverlauf werden zur Answertung graphisch aufgezeichnet.

Zur Gewöhnung an den Meßvorgang werden die Tiere vor Substanzprüfung 14 Tage trainiert. In der zweiten Trainingswoche werden Blutdruck-Vorwerte erhoben. Tiergruppen, die Substanz erhalten, werden gegen eine Kontrollgruppe geprüft.

In der anschließenden Tabelle werden die untersuchten Verbindungen durch laufende Nummern gekennzeichnet, die wie folgt zugeordnet sind:

| lfd. Nr. | Name der Verbindung |
|---|---|
| 1 | 1,4-Dihydro-2,6-dimethyl-4-(3-nitrophenyl)-pyridin-3,5-dicarbonsäure-3-methyl-5-[3-(4,4-diphenylpiperidyl-1)-propyl]-ester-hydrochlorid |
| 2 | 1,4-Dihydro-2,6-dimethyl-4-(3-nitrophenyl)-pyridin-3,5-dicarbonsäure-3-methyl-5-[2-(4,4-diphenylpiperidyl-1)-ethyl]-ester-hydrochlorid |
| 3 | 4-(3-Cyanphenyl)-1,4-dihydro-2,6-dimethylpyridin-3,5-dicarbonsäure-3-methyl-5-[3-(4,4-diphenylpiperidyl-1)-propyl]-ester-hydrochlorid |
| 4 | 1,4-Dihydro-2,6-dimethyl-4-(3-nitrophenyl)-pyridin-3,5-dicarbonsäure-3-methyl-5-[2-(4,4-diphenylpiperidyl-1)-2-methylpropyl]-ester-hydrochlorid |
| 5 | 1,4-Dihydro-2,6-dimethyl-4-(3-nitrophenyl)-pyridin-3,5-dicarbonsäure-3-ethyl-5-[3-(4,4-diphenylpiperidyl-1)-propyl]-ester-hydrochlorid |
| 6 | 1,4-Dihydro-2,6-dimethyl-4-[3-(1,1,2,2-tetrafluorethoxi)-phenyl]-pyridin-3,5-dicarbonsäure-3-methyl-5-[3-(4,4-diphenylpiperidyl-1)-propyl]-ester-hydrochlorid |
| 7 | 1,4-Dihydro-2,6-dimethyl-4-(3-nitrophenyl)-pyridin-3,5-dicarbonsäure-3-(2-methoxiethyl)-5-[2-(4,4-diphenylpiperidyl-1)-ethyl]-ester-fumarat |
| 8 | 1,4-Dihydro-2,6-dimethyl-4-(3-nitrophenyl)-pyridin-3,5-dicarbonsäure-3-(2-methoxiethyl)-5-[3-(4,4-diphenylpiperidyl-1)-propyl]-ester-fumarat |
| 9 | 1,4-Dihydro-2,6-dimethyl-4-(3-nitrophenyl)-pyridin-3,5-dicarbonsäure-3-methyl-5-{3-[4,4-di-(4-methoxiphenyl)-piperidyl-1]-propyl}-ester-hydrochlorid |
| 10 | 1,4-Dihydro-2,6-dimethyl-4-(3-nitrophenyl)-pyridin-3,5-dicarbonsäure-3-methyl-5-[4-(4,4-diphenylpiperidyl-1)-butyl]-ester-fumarat |

| lfd. Nr. | Name der Verbindung |
|---|---|
| 11 | 1,4-Dihydro-2,6-dimethyl-4-(3-nitrophenyl)-pyridin-3,5-dicarbonsäure-3-methyl-5-[1,1-dimethyl-2-(4,4-diphenylpiperidyl-1)-ethyl]-ester-hydrochlorid |
| 12 | 1,4-Dihydro-2,6-dimethyl-4-(2-difluormethoxiphenyl)-pyridin-3,5-dicarbonsäure-3-ethyl-5-[3-(4,4-diphenylpiperidyl-1)-propyl]-ester-hydrochlorid |
| 13 | 1,4-Dihydro-2,6-dimethyl-4-(3-difluormethoxiphenyl)-pyridin-3,5-dicarbonsäure-3-ethyl-5-[3-(4,4-diphenylpiperidyl-1)-propyl]-ester-hydrochlorid |
| 14 | 4-(2,3-Dichlorphenyl)-1,4-dihydro-2,6-dimethylpyridin-3,5--dicarbonsäure-3-methyl-5-[3-(4,4-diphenylpiperidyl-1)-propyl]-ester-hydrochlorid |
| 15 | 4-(2,1,3-Benzoxdiazol-4-yl)-1,4-dihydro-2,6-dimethyl-pyridin-3,5-dicarbonsäure-3-methyl-5-[3-(4,4-diphenylpiperidyl-1)-propyl]-ester-hydrochlorid |
| 16 | 1,4-Dihydro-2,6-dimethyl-4-(3-fluorphenyl)-pyridin-3,5-dicarbonsäure-3-methyl-5-[3-(4,4-diphenylpiperidyl-1)-propyl]-ester-hydrochlorid |
| 17 | 1,4-Dihydro-2,6-dimethyl-4-(2-trifluromethylphenyl)-pyridin-3,5-dicarbonsäure-3-methyl-5-[3-(4,4-diphenylpiperidyl-1)-propyl]-ester-hydrochlorid |
| 18 | 4-(2-Cyanphenyl)-1,4-dihydro-2,6-dimethylpyridin-3,5-dicarbonsäure-3-ethyl-5-[3-(4,4-diphenylpiperidyl-1)-propyl]-ester-hydrochlorid |
| 19 | 4-(2-Chlorphenyl)-1,4-dihydro-2,6-dimethylpyridin-3,5-dicarbonsäure-3-methyl-5-[3-(4,4-diphenylpiperidyl-1)-propyl]-ester-hydrochlorid |

Tabelle I gibt für die Vertreter der erfindungsgemäßen Verbindungen die prozentuale Senkung des Blutdrucks [BP) nach oraler Verabreichung bei der Ratte wieder.

Tabelle I

%-Änderungen (BP) an genetisch hypertonen Ratten nach täglich einmaliger p.o.-Applikation an vier aufeinanderfolgenden Tagen (N=6/Dosis); die Dosis in mg/kg ist auf die freie Base berechnet.

| lfd. Nr. | Dosis µMol/kg | Dosis mg/kg | BP (% Änderung vs. Kontrolle), Mittelwert für Meßzeitpunkte: Stunden nach Gabe (Tage) 2h (1.+4.Tag) | 6h (1.-4.Tag) | 24h (1.+3.Tag) |
|---|---|---|---|---|---|
| 1 | 5 | 3,05 | -47 | -30 | -7 |
| | 10 | 6,09 | -50 | -37 | -9 |
| | 25 | 15,24 | -48 | -45 | -25 |
| 2 | 25 | 14,89 | -51 | -36 | -2 |
| 3 | 25 | 14,74 | -48 | -41 | -19 |
| 4 | 25 | 15,60 | -51 | -44 | -25 |
| 5 | 10 | 6,24 | -51 | -41 | -20 |
| 6 | 25 | 17,02 | -21 | -21 | -10 |
| 7 | 25 | 15,99 | -44 | -33 | 0 |
| 8 | 25 | 16,34 | -48 | -28 | -5 |
| 9 | 10 | 6,70 | -53 | -45 | -14 |
| 10 | 10 | 6,24 | -40 | -18 | -2 |
| 11 | 5 | 3,12 | -54 | -33 | -15 |
| 12 | 25 | 16,12 | -31 | -11 | +4 |
| 13 | 25 | 16,12 | -41 | -29 | -6 |
| 14 | 25 | 15,84 | -49 | -43 | -30 |
| 15 | 25 | 15,17 | -49 | -41 | -16 |
| 16 | 25 | 14,57 | -46 | -38 | -7 |
| 17 | 25 | 15,82 | -44 | -30 | -17 |
| 18 | 25 | 15,09 | -37 | -25 | -1 |
| 19 | 25 | 14,98 | -43 | -28 | -2 |

**Patentansprüche**

**Patentansprüche für folgende Vertragsstaaten : BE, CH, DE, FR, GB, IT, LI, LU, NL, SE**

1. Verbindungen der allgemeinen Formel I

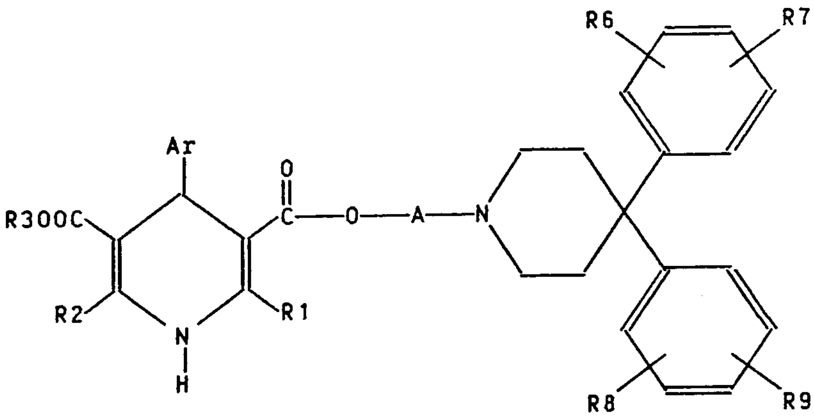

(I),

worin Ar einen Cyclus der Formel

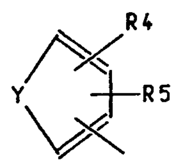

darstellt, in dem Y Sauerstoff (O), Schwefel (S), Vinylen (-CH = CH-), Azomethin (-CH = N-) oder eine Gruppe der Formel

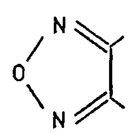

oder

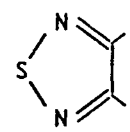

bedeutet,
R1, R2 und R3 gleich oder verschieden sind und Wasserstoff, $C_1$-$C_6$-alkyl oder $C_3$-$C_7$-alkoxyalkyl bedeuten,
R4 und R5 gleich oder verschieden sind und Wasserstoff, Hydroxy, Halogen, Nitro, Cyano, Trifluormethyl, $C_1$-$C_4$-alkyl, $C_1$-$C_4$-alkoxy, ganz oder teilweise durch Fluor substituiertes $C_1$-$C_4$-alkoxy, $C_1$-$C_4$-alkoxycarbonyl, $C_2$-$C_5$-acyl, Amino oder Mono- oder Di-$C_1$-$C_4$-alkylamino bedeuten, R6, R7, R8 und R9 gleich oder verschieden sind und Wasserstoff, Hydroxy, Halogen, $C_1$-$C_4$-alkyl, $C_1$-$C_4$-alkoxy, Amino, Mono- oder Di-$C_1$-$C_4$-alkylamino oder ganz oder teilweise durch Fluor substituiertes $C_1$-$C_4$-alkoxy bedeuten,
A geradkettiges oder verzweigtes $C_2$-$C_5$-alkylen bedeutet,
und ihre Salze.

2. Verbindungen der Formel I nach Anspruch 1, worin Ar den in Anspruch 1 genannten Cyclus darstellt, in dem Y Vinylen (-CH = CH-) bedeutet, und R1, R2, R3, R4, R5, R6, R7, R8, R9 und A die in Anspruch 1 angebenen Bedeutungen haben, und ihre Salze.

3. Verbindungen der Formel I nach Anspruch 1, worin Ar den in Anspruch 1 genannten Cyclus darstellt, in dem Y

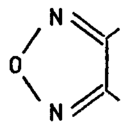

bedeutet, R4 und R5 Wasserstoff bedeuten und R1, R2, R3, R6, R7, R8, R9 und A die in Anspruch 1 angebenen Bedeutungen haben, und ihre Salze.

**4.** Verbindungen nach einem der Ansprüche 1 bis 3, worin R1 $C_1$-$C_4$-alkyl, R2 $C_1$-$C_4$-alkyl, R3 $C_1$-$C_4$-alkyl oder $C_3$-$C_5$-alkoxyalkyl, R4 Wasserstoff, Chlor, Nitro, Methyl oder Methoxy, R5 Wasserstoff, Chlor, Fluor, Nitro, Cyano, Methyl, Methoxy, Difluormethoxy, 1,1,2,2-Tetrafluorethoxy, Trifluormethyl oder Acetyl, R6 Wasserstoff oder Methoxy, R7 Wasserstoff, Hydroxy, Chlor, Methyl, Methoxy, Difluormethoxy oder 1,1,2,2-Tetrafluorethoxy, R8 Wasserstoff oder Methoxy, R9 Wasserstoff, Hydroxy, Chlor, Methyl, Methoxy, Difluormethoxy oder 1,1,2,2-Tetrafluorethoxy und A Ethylen oder Propylen bedeutet, und ihre Salze.

**5.** Verbindungen nach einem der Ansprüche 1 bis 3, worin R1 Methyl, R2 Methyl, R3 Methyl, Ethyl oder Methoxyethyl, R4 Wasserstoff, R5 Wasserstoff, Chlor, Fluor, Nitro, Cyano, Methyl, Methoxy, Difluormethoxy, 1,1,2,2-Tetrafluorethoxy oder Trifluormethyl, R6 Wasserstoff, R7 Wasserstoff, Hydroxy, Chlor, Methyl, Methoxy, Difluormethoxy oder 1,1,2,2-Tetrafluorethoxy, R8 Wasserstoff, R9 Waserstoff, Hydroxy, Chlor, Methyl, Methoxy, Difluormethoxy oder 1,1,2,2-Tetrafluorethoxy und A Ethylen oder Propylen bedeutet, und ihre Salze.

**6.** Verbindungen nach Anspruch 2, worin R1 Methyl, R2 Methyl, R3 Methyl, Ethyl oder Methoxyethyl, R4 Wasserstoff oder Chlor, R5 Wasserstoff, Chlor, Fluor, Nitro, Cyano, Difluormethoxy, 1,1,2,2-Tetrafluorethoxy oder Trifluormethyl, R6 Wasserstoff, R7 Wasserstoff oder Methoxy, R8 Wasserstoff, R9 Wasserstoff oder Methoxy und A Ethylen, Propylen, Butylen, 1,1-Dimethylethylen oder 2,2-Dimethylethylen bedeutet, und ihre Salze.

**7.** Verbindungen nach Anspruch 1, worin Ar Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl oder 3-Trifluormethylphenyl, R1 Methyl, R2 Methyl, R3 Methyl, Ethyl oder Methoxyethyl, R6 Wasserstoff, R7 Wasserstoff oder Methoxy, R8 Wasserstoff, R9 Wasserstoff oder Methoxy und A Ethylen, Propylen, Butylen, 1,1-Dimethylethylen oder 2,2-Dimethylethylen bedeutet, und ihre Salze.

**8.** Verbindungen nach Anspruch 3, worin R1 Methyl, R2 Methyl, R3 Methyl, Ethyl oder Methoxyethyl, R4 Wasserstoff, R5 Wasserstoff, R6 Wasserstoff, R7 Wasserstoff oder Methoxy, R8 Wasserstoff, R9 Wasserstoff oder Methoxy, und A Ethylen, Propylen, Butylen, 1,1-Dimethylethylen oder 2,2-Dimethylethylen bedeutet, und ihre Salze.

**9.** Verbindungen nach Anspruch 1, worin Ar die Bedeutung 2-Nitrophenyl oder 3-Nitrophenyl hat, R1, R2 und R3 gleich oder verschieden sind und die Bedeutung $C_1$-$C_6$-alkyl oder $C_3$-$C_7$-alkoxyalkyl haben, A geradkettiges oder verzweigtes $C_2$-$C_5$-alkylen darstellt und R6, R7, R8 und R9 Wasserstoff bedeuten, und ihre Salze.

**10.** Verbindungen nach Anspruch 1, worin Ar 2-Nitrophenyl oder 3-Nitrophenyl, R1 Methyl, R2 Methyl, R3 Methyl, Ethyl oder Methoxyethyl, R6 Wasserstoff, R7 Wasserstoff, R8 Wasserstoff, R9 Wasserstoff und A Ethylen oder Propylen bedeutet, und ihre Salze.

**11.** Verbindung ausgewählt aus der Gruppe bestehend aus
1,4-Dihydro-2,6-dimethyl-4-(3-nitrophenyl)-pyridin-3,5-dicarbonsäure-3-methyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-methyl-5-[2-(4,4-diphenylpiperidyl-1)-ethyl]-ester,
4-(3-Cyanphenyl)-1,4-dihydro-2,6-dimethylpyridin-3,5-dicarbonsäure-3-methyl-5-[3-(4,4-diphenylpiperidyl-1)-propyl]-ester,
1,4-Dihydro-2,6-dimethyl-4-(3-nitrophenyl)-pyridin-3,5-dicarbonsäure-3--methyl-5-[2-(4,4-diphenylpiperidyl-1)-2-methyl-propyl]-ester,
1,4-Dihydro-2,6-dimethyl-4-(3-nitrophenyl)-pyridin-3,5-dicarbonsäure-3-ethyl-5-[3-(4,4-diphenylpiperidyl-1)-propyl]-ester,
1,4-Dihydro-2,6-dimethyl-4-[3-(1,1,2,2-tetrafluorethoxi)-phenyl]-pyridin-3,5-dicarbonsäure-3-methyl-5-[3-4,4-diphenylpiperidyl-1)-propyl]-ester,
1,4-Dihydro-2,6-dimethyl-4-[3-(1,1,2,2-tetrafluorethoxi)-phenyl]-pyridin-3,5-dicarbonsäure-3-methyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(2-methoxiethyl)-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(2-methoxiethyl)-5-[3-(4,4-diphenylpiperidyl-1)-propyl]-ester,
1,4-Dihydro-2,6-dimethyl-4-(3-nitrophenyl)-pyridin-3,5-dicarbonsäure-3-methyl-5-{3-[4,4-di-(4-methoxiphenyl)-piperidyl-1]-propyl}-ester,
1,4-Dihydro-2,6-dimethyl-4-(3-nitrophenyl)-pyridin-3,5-dicarbonsäure-3-methyl-5-[4-(4,4-diphenylpiperidyl-1)-butyl]-ester,
1,4-Dihydro-2,6-dimethyl-4-(3-nitrophenyl)-pyridin-3,5-dicarbonsäure-3-methyll-5-[1,1-dimethyl-2-(4,4-diphenylpiperidyl-1)-ethyl]ester,
1,4-Dihydro-2,6-dimethyl-4-(2-difluormethoxiphenyl)-pyridin-3,5-dicarbonsäure-3-ethyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-[3-(4,4-diphenylpiperidyl-1)-propyl]-ester,
1,4-Dihydro-2,6-dimethyl-4-[3-(1,1,2,2-tetrafluorethoxi)-phenyl]-pyridin-3,5-dicarbonsäure-3-ethyl-5-[3-(4,4-diphenylpiperidyl-1)-propyl]-ester,
1,4-Dihydro-2,6-dimethyl-4-(3-difluormethoxiphenyl)-pyridin-3,5-dicarbonsäure-3-ethyl-5-[3-(4,4-diphenylpiperidyl-1)-propyl]-ester,
4-(2,3-Dichlorphenyl)-1,4-dihydro-2,6-dimethylpyridin-3,5-dicarbonsäure-3-methyl-5-[3-(4,4-diphenylpiperidyl-1)-propyl]-ester,
4-(2,1,3-Benzoxdiazol-4-yl)-1,4-dihydro-2,6-dimethyl-pyridin-3,5-dicarbonsäure-3-methyl-5-[3-(4,4-diphenylpiperidyl-1)-propyl]-ester,
1,4-Dihydro-2,6-dimethyl-4-(3-fluorphenyl)-pyridin-3,5-dicarbonsäure-3-methyl-5-[3-(4,4-diphenylpiperidyl-1)-propyl]-ester,
1,4-Dihydro-2,6-dimethyl-4-(2-trifluromethylphenyl)-pyridin-3,5-dicarbonsäure-3-methyl-5-[3-(4,4-diphenylpiperidyl-1)-propyl]-ester,
4-(2-Cyanphenyl)-1,4-dihydro-2,6-dimethylpyridin-3,5-dicarbonsäure-3-ethyl-5-[3-(4,4-diphenylpiperidyl-1)-propyl]-ester,
4-(2-Chlorphenyl)1,4-dihydro-2,6-dimethylpyridin-3,5-dicarbonsäure-3-methyl-5-[3-(4,4-diphenylpiperidyl-1)-propyl]-ester-hydrochlorid und ihren Salzen.

**12.** 1,4-Dihydro-2,6-dimethyl-4-(3-nitrophenyl)-pyridin-3,5-dicarbonsäure-3-methyl-5-[3-(4,4-diphenylpiperidyl-1)-propyl]-ester und seine Salze.

**13.** Verfahren zur Herstellung von Verbindungen der Formel I nach Anspruch 1 und ihren Salzen, dadurch gekennzeichnet, daß man

a) Zimtsäurederivate der Formel II

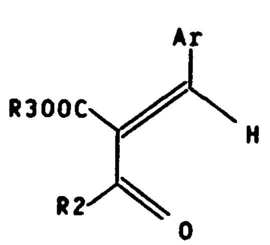

(II),

mit Enaminderivaten der Formel III

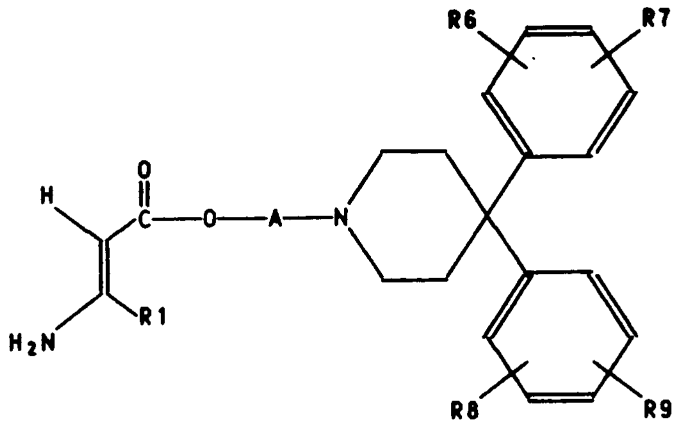

(III),

oder

b) Zimtsäurederivate der Formel II mit Ammoniak und β-Ketocarbonsäurederivaten der Formel IV

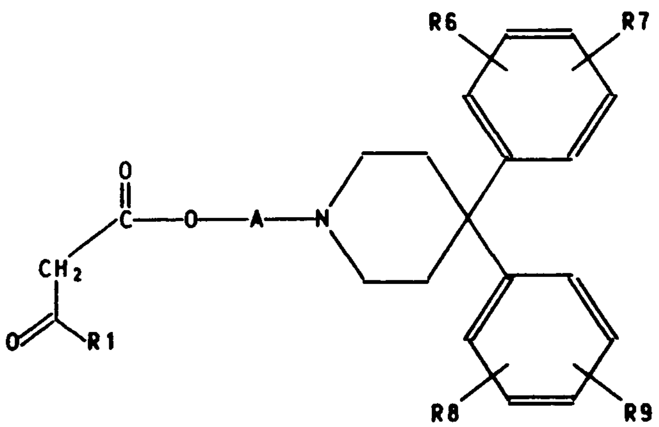

(IV),

oder

c) Enamine der Formel V

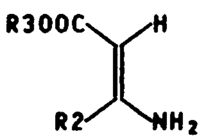

(V).

mit Benzylidencarbonsäurederivaten der Formel VI

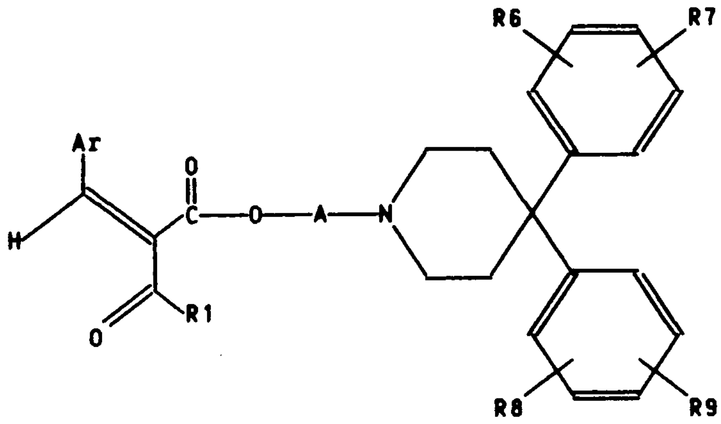

(VI),

oder

d) Ketoverbindungen der Formel VII

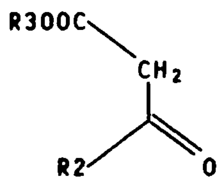

(VII),

mit Ammoniak und Benzylidencarbonsäurederivaten der Formel VI, oder

e) Aldehyde der Formel VIII

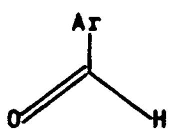

(VIII),

mit Enaminen der Formel V und $\beta$-Ketocarbonsäurederivaten der Formel IV, oder

f) Aldehyde der Formel VIII mit Enaminderivaten der Formel III und Ketoverbindungen der Formel VII, oder

g) 1,4-Dihydropyridine der Formel IX

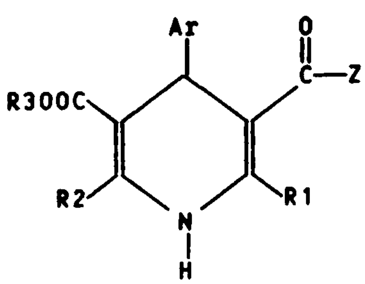

(IX),

mit Diarylverbindungen der Formel X

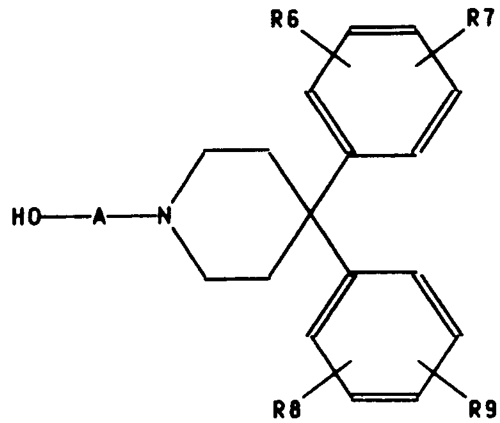

(X),

als solche(n) oder in Form ihrer Salze umsetzt und gewünschtenfalls anschließend erhaltene Salze in die freien Basen oder erhaltene Basen in die Salze überführt, wobei Ar, R1, R2, R3, R4, R5, R6, R7, R8, R9, Y und A die in Anspruch 1 angegebenen Bedeutungen haben und Z gemeinsam mit der Carbonylgruppe, woran es gebunden ist, eine Carboxylgruppe oder ein reaktives Carbonsäurederivat darstellt.

**14.** Arzneimittel enthaltend eine oder mehrere Verbindungen nach einem oder mehreren der Ansprüche 1 bis 12 und/oder ihre pharmakologisch verträglichen Salze.

**15.** Verbindungen nach einem oder mehreren der Ansprüche 1 bis 12 und ihre pharmakologisch verträglichen Salze zur Anwendung bei der Behandlung und/oder Prophylaxe von Hypertonie, koronaren Herzkrankheiten, peripheren und cerebralen Zirkulationsstörungen und/oder Krankheiten, die auf einer erhöhten Wasser- oder Natriumretention beruhen.

**16.** Verwendung von Verbindung nach einem oder mehreren der Ansprüche 1 bis 12 und ihren pharmakologisch verträglichen Salzen zur Herstellung von Arzneimitteln für die Behandlung und/oder Prophylaxe von Hypertonie, koronaren Herzkrankheiten, peripheren und cerebralen Zirkulationsstörungen und/oder Krankheiten, die auf einer erhöhten Wasser- oder Natriumretention beruhen.

**Patentansprüche für folgenden Vertragsstaat : AT**

**1.** Verfahren zur Herstellung von Verbindungen der allgemeinen Formel I

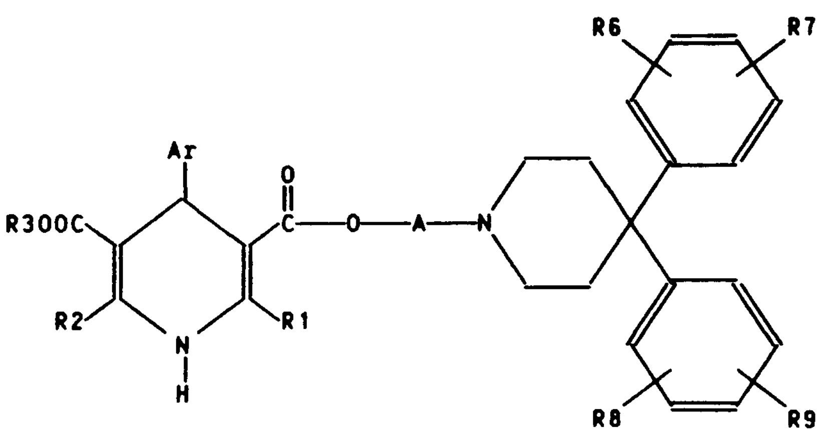

(I),

worin Ar einen Cyclus der Formel

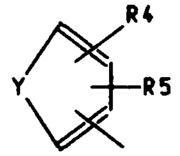

darstellt, in dem Y Sauerstoff (O), Schwefel (S), Vinylen (-CH = CH-), Azomethin (-CH = N-) oder eine Gruppe der Formel

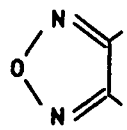

oder

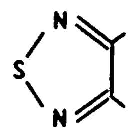

bedeutet,
R1, R2 und R3 gleich oder verschieden sind und Wasserstoff, $C_1$-$C_6$-alkyl oder $C_3$-$C_7$-alkoxyalkyl bedeuten,
R4 und R5 gleich oder verschieden sind und Wasserstoff, Hydroxy, Halogen, Nitro, Cyano, Trifluormethyl, $C_1$-$C_4$-alkyl, $C_1$-$C_4$-alkoxy, ganz oder teilweise durch Fluor substituiertes $C_1$-$C_4$-alkoxy, $C_1$-$C_4$-alkoxycarbonyl, $C_2$-$C_5$-acyl, Amino oder Mono- oder Di-$C_1$-$C_4$-alkylamino bedeuten, R6, R7, R8 und R9 gleich oder verschieden sind und Wasserstoff, Hydroxy, Halogen, $C_1$-$C_4$-alkyl, $C_1$-$C_4$-alkoxy, Amino, Mono- oder Di-$C_1$-$C_4$-alkylamino oder ganz oder teilweise durch Fluor substituiertes $C_1$-$C_4$-alkoxy bedeuten,
A geradkettiges oder verzweigtes $C_2$-$C_5$-alkylen bedeutet
und ihren Salzen, dadurch gekennzeichnet, daß man

a) Zimtsäurederivate der Formel II

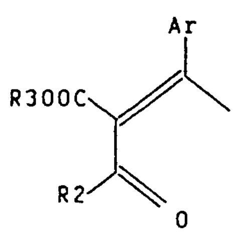

(II),

mit Enaminderivaten der Formel III

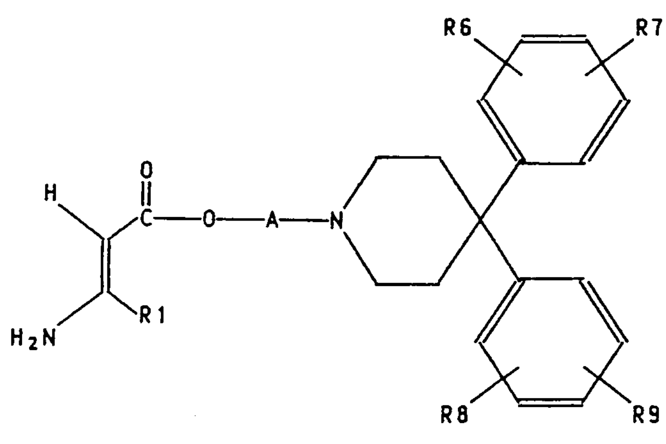

(III),

oder

b) Zimtsäurederivate der Formel II mit Ammoniak und $\beta$-Ketocarbonsäurederivaten der Formel IV

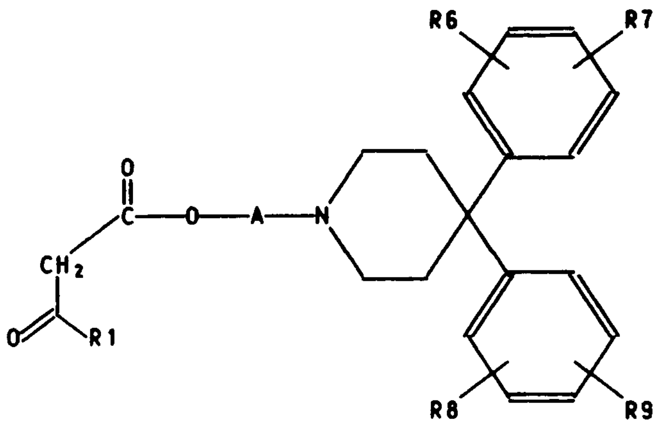

(IV),

oder
c) Enamine der Formel V

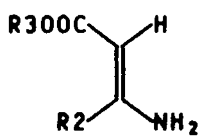

(V).

mit Benzylidencarbonsäurederivaten der Formel VI

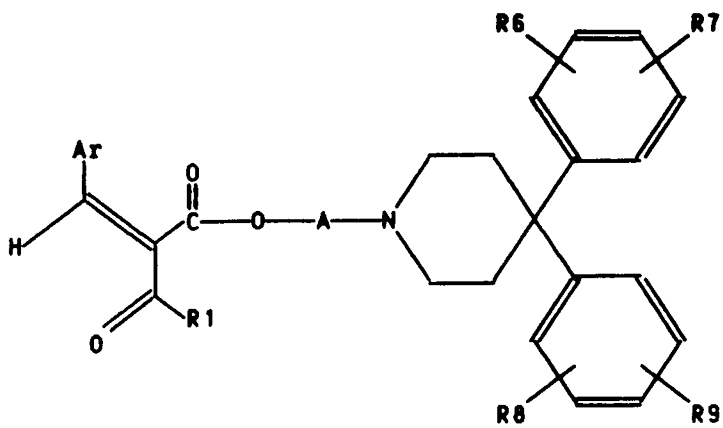

(VI),

oder
d) Ketoverbindungen der Formel VII

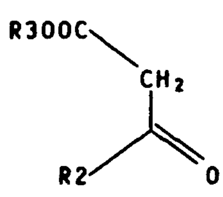

(VII),

mit Ammoniak und Benzylidencarbonsäurederivaten der Formel VI, oder

e) Aldehyde der Formel VIII

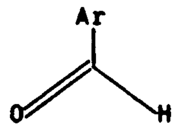

(VIII),

mit Enaminen der Formel V und β-Ketocarbonsäurederivaten der Formel IV, oder

f) Aldehyde der Formel VIII mit Enaminderivaten der Formel III und Ketoverbindungen der Formel VII, oder

g) 1,4-Dihydropyridine der Formel IX

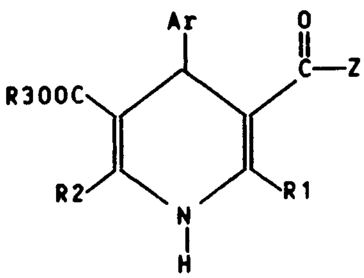

(IX),

mit Diarylverbindungen der Formel X

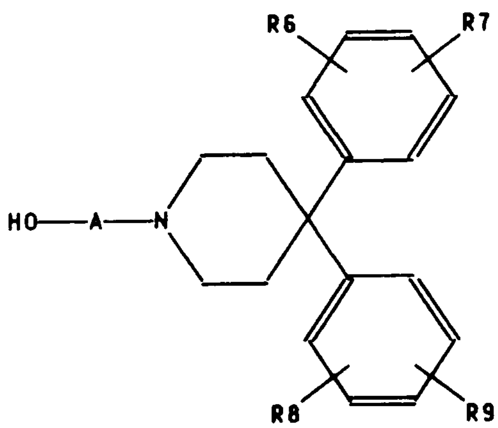

(X),

als solche(n) oder in Form ihrer Salze umsetzt und gewünschtenfalls anschließend erhaltene Salze in die freien Basen oder erhaltene Basen in die Salze überführt, wobei Ar, R1, R2, R3, R4, R5, R6, R7, R8, R9, Y und A die oben angegebenen Bedeutungen haben und Z gemeinsam mit der Carbonylgruppe, woran es gebunden ist, eine Carboxylgruppe oder ein reaktives Carbonsäurederivat darstellt.

**2.** Verfahren nach Anspruch 1, worin Ar den in Anspruch 1 genannten Cyclus darstellt, in dem Y Vinylen (-CH=CH-) bedeutet, und R1, R2, R3, R4, R5, R6, R7, R8, R9 und A die in Anspruch 1 angebenen Bedeutungen haben.

**3.** Verfahren nach Anspruch 1, worin Ar den in Anspruch 1 genannten Cyclus darstellt, in dem Y

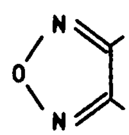

bedeutet, R4 und R5 Wasserstoff bedeuten und R1, R2, R3, R6, R7, R8, R9 und A die in Anspruch 1 angebenen Bedeutungen haben.

**4.** Verfahren nach einem der Ansprüche 1 bis 3, worin R1 $C_1$-$C_4$-alkyl, R2 $C_1$-$C_4$-alkyl, R3 $C_1$-$C_4$-alkyl oder $C_3$-$C_5$-alkoxyalkyl, R4 Wasserstoff, Chlor, Nitro, Methyl oder Methoxy, R5 Wasserstoff, Chlor, Fluor, Nitro, Cyano, Methyl, Methoxy, Difluormethoxy, 1,1,2,2-Tetrafluorethoxy, Trifluormethyl oder Acetyl, R6 Wasserstoff oder Methoxy, R7 Wasserstoff, Hydroxy, Chlor, Methyl, Methoxy, Difluormethoxy oder 1,1,2,2-Tetrafluorethoxy, R8 Wasserstoff oder Methoxy, R9 Wasserstoff, Hydroxy, Chlor, Methyl, Methoxy, Difluormethoxy oder 1,1,2,2-Tetrafluorethoxy und A Ethylen oder Propylen bedeutet.

**5.** Verfahren nach einem der Ansprüche 1 bis 3, worin R1 Methyl, R2 Methyl, R3 Methyl, Ethyl oder Methoxyethyl, R4 Wasserstoff, R5 Wasserstoff, Chlor Fluor, Nitro, Cyano, Methyl, Methoxy, Difluormethoxy, 1,1,2,2-Tetrafluorethoxy oder Trifluormethyl, R6 Wasserstoff, R7 Wasserstoff, Hydroxy, Chlor, Methyl, Methoxy, Difluormethoxy oder 1,1,2,2-Tetrafluorethoxy, R8 Wasserstoff, R9 Wasserstoff, Hydroxy, Chlor, Methyl, Methoxy, Difluormethoxy oder 1,1,2,2-Tetrafluorethoxy und A Ethylen oder Propylen bedeutet.

**6.** Verfahren nach Anspruch 2, worin R1 Methyl, R2 Methyl, R3 Methyl, Ethyl oder Methoxyethyl, R4 Wasserstoff oder Chlor, R5 Wasserstoff, Chlor Fluor, Nitro, Cyano, Difluormethoxy, 1,1,2,2-Tetrafluorethoxy oder Trifluormethyl, R6 Wasserstoff, R7 Wasserstoff oder Methoxy, R8 Wasserstoff, R9 Wasserstoff oder Methoxy und A Ethylen, Propylen, Butylen, 1,1-Dimethylethylen oder 2,2-Dimethylethylen bedeutet.

**7.** Verfahren nach Anspruch 1, worin Ar Phenyl, 2-Nitrophenyl, 3-Nitrophenyl, 2-Cyanophenyl, 3-Cyanophenyl, 2-(1,1,2,2-Tetrafluorethoxy)-phenyl, 3-(1,1,2,2-Tetrafluorethoxy)-phenyl, 2-Difluormethoxyphenyl, 3-Difluormethoxyphenyl, 2-Chlorphenyl, 3-Chlorphenyl, 2,3-Dichlorphenyl, 2-Fluorphenyl, 3-Fluorphenyl, 2-Trifluormethylphenyl oder 3-Trifluormethylphenyl, R1 Methyl, R2 Methyl, R3 Methyl, Ethyl oder Methoxyethyl, R6 Wasserstoff, R7 Wasserstoff oder Methoxy, R8 Wasserstoff, R9 Wasserstoff oder Methoxy und A Ethylen, Propylen, Butylen, 1,1-Dimethylethylen oder 2,2-Dimethylethylen bedeutet.

**8.** Verfahren nach Anspruch 3, worin R1 Methyl, R2 Methyl, R3 Methyl, Ethyl oder Methoxyethyl, R4 Wasserstoff, R5 Wasserstoff, R6 Wasserstoff, R7 Wasserstoff oder Methoxy, R8 Wasserstoff, R9 Wasserstoff oder Methoxy, und A Ethylen, Propylen, Butylen, 1,1-Dimethylethylen oder 2,2-Dimethylethylen bedeutet.

**9.** Verfahren nach Anspruch 1, worin Ar die Bedeutung 2-Nitrophenyl oder 3-Nitrophenyl hat, R1, R2 und R3 gleich oder verschieden sind und die Bedeutung $C_1$-$C_6$-alkyl oder $C_3$-$C_7$-alkoxyalkyl haben, A geradkettiges oder verzweigtes $C_2$-$C_5$-alkylen darstellt und R6, R7, R8 und R9 Wasserstoff bedeuten.

**10.** Verfahren nach Anspruch 1, worin Ar 2-Nitrophenyl oder 3-Nitrophenyl, R1 Methyl, R2 Methyl, R3 Methyl, Ethyl oder Methoxyethyl, R6 Wasserstoff, R7 Wasserstoff, R8 Wasserstoff, R9 Wasserstoff und A Ethylen oder Propylen bedeutet.

**11.** Verfahren nach Anspruch 1, dadurch gekennzeichnet, daß eine Verbindung ausgewählt aus der Gruppe bestehend aus
1,4-Dihydro-2,6-dimethyl-4-(3-nitrophenyl)-pyridin-3,5-dicarbonsäure-3-methyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-methyl-5-[2-(4,4-diphenylpiperidyl-1)-ethyl]-ester,
4-(3-Cyanphenyl)-1,4-dihydro-2,6-dimethylpyridin-3,5-dicarbonsäure-3-methyl-5-[3-(4,4-diphenylpiperidyl-1)-propyl]-ester,
1,4-Dihydro-2,6-dimethyl-4-(3-nitrophenyl)-pyridin-3,5-dicarbonsäure-3--methyl-5-[2-(4,4-diphenylpiperidyl-1)-2-methyl-propyl]-ester,
1,4-Dihydro-2,6-dimethyl-4-(3-nitrophenyl)-pyridin-3,5-dicarbonsäure-3-ethyl-5-[3-(4,4-diphenylpiperidyl-1)-propyl]-ester,
1,4-Dihydro-2,6-dimethyl-4-[3-(1,1,2,2-tetrafluorethoxi)-phenyl]-pyridin-3,5-dicarbonsäure-3-methyl-5-[3-(4,4-diphenylpiperidyl-1)-propyl]-ester,
1,4-Dihydro-2,6-dimethyl-4-[3-(1,1,2,2-tetrafluorethoxi)-phenyl]-pyridin-3,5-dicarbonsäure-3-methyl-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(2-methoxiethyl)-5-[2-(4,4-diphenylpiperidyl-1)-ethyl]-ester,
1,4-Dihydro-2,6-dimethyl-4-(3-nitrophenyl)-pyridin-3,5-dicarbonsäure-3-(2-methoxiethyl)-5-[3-(4,4-diphenylpiperidyl-1)-propyl]-ester,
1,4-Dihydro-2,6-dimethyl-4-(3-nitrophenyl)-pyridin-3,5-dicarbonsäure-3-methyl-5-{3-[4,4-di-(4-methoxiphenyl)-piperidyl-1]-propyl}-ester,
1,4-Dihydro-2,6-dimethyl-4-(3-nitrophenyl)-pyridin-3,5-dicarbonsäure-3-methyl-5-[4-(4,4-diphenylpiperidyl-1)-butyl]-ester,
1,4-Dihydro-2,6-dimethyl-4-(3-nitrophenyl)-pyridin-3,5-dicarbonsäure-3-methyl-5-[1,1-dimethyl-2-(4,4-diphenylpiperidyl-1)-ethyl]ester,
1,4-Dihydro-2,6-dimethyl-4-(2-difluormethoxiphenyl)-pyridin-3,5-dicarbonsäure-3-ethyl-5-[3-(4,4-diphenylpiperidyl-1)-propyl]-ester,
1,4-Dihydro-2,6-dimethyl-4-(3-nitrophenyl)-pyridin-3,5-dicarbonsäure-3-[3-(4,4-diphenylpiperidyl-1)-propyl]-ester,
1,4-Dihydro-2,6-dimethyl-4-[3-(1,1,2,2-tetrafluorethoxi)-phenyl]-pyridin-3,5-dicarbonsäure-3-ethyl-5-[3-(4,4-diphenylpiperidyl-1)-propyl]-ester,
1,4-Dihydro-2,6-dimethyl-4-(3-difluormethoxiphenyl)-pyridin-3,5-dicarbonsäure-3-ethyl-5-[3-(4,4-diphenylpiperidyl-1)-propyl]-ester,
4-(2,3-Dichlorphenyl)-1,4-dihydro-2,6-dimethylpyridin-3,5-dicarbonsäure-3-methyl-5-[3-(4,4-diphenylpiperidyl-1)-propyl]-ester,
4-(2,1,3-Benzoxdiazol-4-yl)-1,4-dihydro-2,6-dimethyl-pyridin-3,5-dicarbonsäure-3-methyl-5-[3-(4,4-diphenylpiperidyl-1)-propyl]-ester,
1,4-Dihydro-2,6-dimethyl-4-(3-fluorphenyl)-pyridin-3,5-dicarbonsäure-3-methyl-5-[3-(4,4-diphenylpiperidyl-1)-propyl]-ester,
1,4-Dihydro-2,6-dimethyl-4-(2-trifluromethylphenyl)-pyridin-3,5-dicarbonsäure-3-methyl-5-[3-(4,4-diphenylpiperidyl-1)-propyl]-ester,
4-(2-Cyanphenyl)-1,4-dihydro-2,6-dimethylpyridin-3,5-dicarbonsäure-3-ethyl-5-[3-(4,4-diphenylpiperidyl-1)-propyl]-ester,
4-(2-Chlorphenyl)1,4-dihydro-2,6-dimethylpyridin-3,5-dicarbonsäure-3-methyl-5-[3-(4,4-diphenylpiperidyl-1)-propyl]-ester-hydrochlorid und ihren Salzen hergestellt wird.

**12.** Verfahren nach Anspruch 1, dadurch gekennzeichnet, daß 1,4-Dihydro-2,6-dimethyl-4-(3-nitrophenyl)-pyridin-3,5-dicarbonsäure-3-methyl-5-[3-(4,4-diphenylpiperidyl-1)-propyl]-ester oder ein Salz davon hergestellt wird.

**13.** Verfahren zur Herstellung von Arzneimitteln, dadurch gekennzeichnet, daß man eine oder mehrere Verbindungen der Formel I, hergestellt nach dem Verfahren des Anspruchs 1, und/oder ihre pharmakologisch verträglichen Salze mit einem pharmazeutischen Hilfs- und/oder Trägerstoff vermischt.

**14.** Verwendung von Verbindungen der Formel I und/oder ihren pharmakologisch verträglichen Salzen, zur Herstellung von Arzneimitteln für die Behandlung und/oder Prophylaxe von Hypertonie, koronaren Herzkrankheiten, peripheren und cerebralen Zirkulationsstörungen und/oder Krankheiten, die auf einer erhöhten Wasser- oder Natriumretention beruhen.

## Claims

### Claims for the following Contracting States : BE, CH, DE, FR, GB, IT, LI, LU, NL, SE

**1.** Compounds of the general formula I

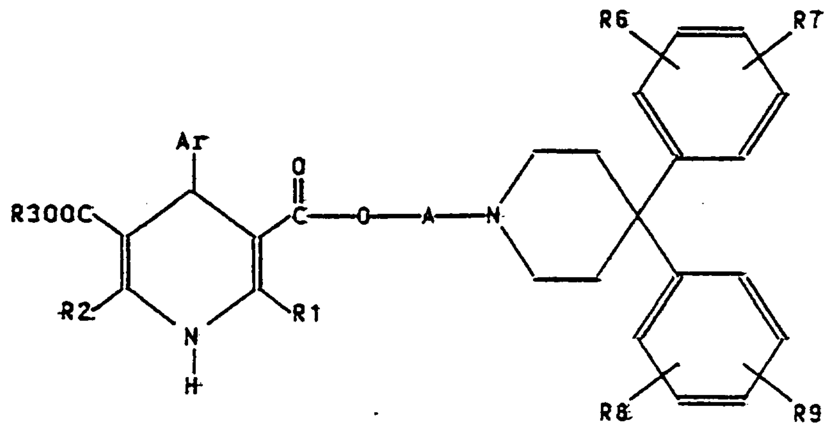

(I)

wherein Ar represents a ring of the formula

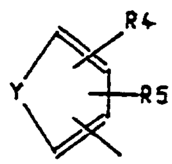

in which Y denotes oxygen (O), sulphur (S), vinylene (-CH = CH-), azomethine (-CH = N-) or a group of the formula

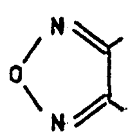

or

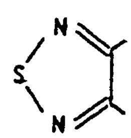

R1, R2 and R3 are identical or different and denote hydrogen, $C_1$-$C_6$-alkyl or $C_3$-$C_7$-alkoxyalkyl,
R4 and R5 are identical or different and denote hydrogen, hydroxyl, halogen, nitro, cyano, trifluoromethyl, $C_1$-$C_4$-alkyl, $C_1$-$C_4$-alkoxy, $C_1$-$C_4$-alkoxy which is completely or partly substituted by fluorine, $C_1$-$C_4$-alkoxycarbonyl, $C_2$-$C_5$-acyl, amino or mono- or di-$C_1$-$C_4$-alkylamino,
R6, R7, R8 and R9 are identical or different and denote hydrogen, hydroxyl, halogen, $C_1$-$C_4$-alkyl, $C_1$-$C_4$-alkoxy, amino, mono- or di-$C_1$-$C_4$-alkylamino, or $C_1$-$C_4$-alkoxy which is completely or partly substituted by fluorine, and
A denotes straight-chain or branched $C_2$-$C_5$-alkylene, and their salts.

**2.** Compounds of the formula I according to Claim 1, wherein Ar represents the ring mentioned in Claim 1, in which Y denotes vinylene (-CH = CH-), and R1, R2, R3, R4, R5, R6, R7, R8, R9 and A have the meanings given in Claim 1, and their salts.

**3.** Compounds of the formula I according to Claim 1, wherein Ar represents the ring mentioned in Claim 1, in which Y denotes

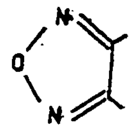

R4 and R5 denote hydrogen and R1, R2, R3, R6, R7, R8, R9 and A have the meanings given in Claim 1, and their salts.

**4.** Compounds according to one of Claims 1 to 3, wherein R1 denotes $C_1$-$C_4$-alkyl, R2 denotes $C_1$-$C_4$-alkyl, R3 denotes $C_1$-$C_4$-alkyl or $C_3$-$C_5$-alkoxyalkyl, R4 denotes hydrogen, chlorine, nitro, methyl or methoxy, R5 denotes hydrogen, chlorine, fluorine, nitro, cyano, methyl, methoxy, difluoromethoxy, 1,1,2,2-tetrafluoroethoxy, trifluoromethyl or acetyl, R6 denotes hydrogen or methoxy, R7 denotes hydrogen, hydroxyl, chlorine, methyl, methoxy, difluoromethoxy or 1,1,2,2-tetrafluoroethoxy, R8 denotes hydrogen or methoxy, R9 denotes hydrogen, hydroxyl, chlorine, methyl, methoxy, difluoromethoxy or 1,1,2,2-tetrafluoroethoxy and A denotes ethylene or propylene, and their salts.

**5.** Compounds accordlng to one of Claims 1 to 3, wherein R1 denotes methyl, R2 denotes methyl, R3 denotes methyl, ethyl or methoxyethyl, R4 denotes hydrogen, R5 denotes hydrogen, chlorine, fluorine, nitro, cyano, methyl, methoxy, difluoromethoxy, 1,1,2,2-tetrafluoroethoxy or trifluoromethyl, R6 denotes hydrogen, R7 denotes hydrogen, hydroxyl, chlorine, methyl, methoxy, difluoromethoxy or 1,1,2,2-tetrafluoroethoxy, R8 denotes hydrogen, R9 denotes hydrogen, hydroxyl, chlorine, methyl, methoxy, difluoromethoxy or 1,1,2,2-tetrafluoroethoxy and A denotes ethylene or propylene, and their salts.

**6.** Compounds according to Claim 2, wherein R1 denotes methyl, R2 denotes methyl, R3 denotes methyl, ethyl or methoxyethyl, R4 denotes hydrogen or chlorine, R5 denotes hydrogen, chlorine, fluorine, nitro, cyano, difluoromethoxy, 1,1,2,2-tetrafluoroethoxy or trifluoromethyl, R6 denotes hydrogen, R7 denotes hydrogen or methoxy, R8 denotes hydrogen, R9 denotes hydrogen or methoxy and A denotes ethylene, propylene, butylene, 1,1-dimethylethylene or 2,2-dimethylethylene, and their salts.

**7.** Compounds according to Claim 1, wherein Ar denotes phenyl, 2-nitrophenyl, 3-nitrophenyl, 2-cyanophenyl, 3-cyanophenyl, 2-(1,1,2,2-tetrafluoroethoxy)-phenyl, 3-(1,1,2,2-tetrafluoroethoxy)-phenyl, 2-difluoromethoxyphenyl, 3-difluoromethoxyphenyl, 2-chlorophenyl, 3-chlorophenyl, 2,3-dichlorophenyl, 2-fluorophenyl, 3-fluorophenyl, 2-trifluoromethylphenyl or 3-trifluoromethylphenyl, R1 denotes methyl, R2 denotes methyl, R3 denotes methyl, ethyl or methoxyethyl, R6 denotes hydrogen, R7 denotes hydrogen or methoxy, R8 denotes hydrogen, R9 denotes hydrogen or methoxy and A denotes ethylene, propylene, butylene, 1,1-dimethylethylene or 2,2-dimethylethylene, and their salts.

**8.** Compounds according to Claim 3, wherein R1 denotes methyl, R2 denotes methyl, R3 denotes methyl, ethyl or methoxyethyl, R4 denotes hydrogen, R5 denotes hydrogen, R6 denotes hydrogen, R7 denotes hydrogen or methoxy, R8 denotes hydrogen, R9 denotes hydrogen or methoxy and A denotes ethylene, propylene, butylene, 1,1-dimethylethylene or 2,2-dimethylethylene, and their salts.

**9.** Compounds according to Claim 1, wherein Ar denotes 2-nitrophenyl or 3-nitrophenyl, R1, R2 and R3 are identical or different and denote $C_1$-$C_6$-alkyl or $C_3$-$C_7$-alkoxyalkyl, A represents straight-chain or branched $C_2$-$C_5$-alkylene and R6, R7, R8 and R9 denote hydrogen, and their salts.

**10.** Compounds according to Claim 1, wherein Ar denotes 2-nitrophenyl or 3-nitrophenyl, R1 denotes methyl, R2 denotes methyl, R3 denotes methyl, ethyl or methoxyethyl, R6 denotes hydrogen, R7 denotes hydrogen, R8 denotes hydrogen, R9 denotes hydrogen and A denotes ethylene or propylene, and their salts.

**11.** Compound selected from the group consisting of
3-methyl 5-[3-(4,4-diphenylpiperid-1-yl)-propyl] 1,4-dihydro-2,6-dimethyl-4-(3-nitrophenyl)-pyridine-3,5-dicarboxylate,
3-methyl 5-[2-(4,4-diphenylpiperid-1-yl)-ethyl] 1,4-dihydro-2,6-dimethyl-4-(3-nitrophenyl)-pyridine-3,5-dicarboxylate,
3-methyl 5-[3-(4,4-diphenylpiperid-1-yl)-propyl] 4-(3-cyanophenyl)-1,4-dihydro-2,6-dimethylpyridine-3,5-dicarboxylate,
3-methyl 5-[2-(4,4-diphenylpiperid-1-yl)-2-methyl-propyl] 1,4-dihydro-2,6-dimethyl-4-(3-nitrophenyl)-pyridine-3,5-dicarboxylate,
3-ethyl 5-[3-(4,4-diphenylpiperid-1-yl)-propyl] 1,4-dihydro-2,6-dimethyl-4-(3-nitrophenyl)-pyridine-3,5-dicarboxylate,
3-methyl 5-[3-(4,4-diphenylpiperid-1-yl)-propyl] 1,4-dihydro-2,6-dimethyl-4-[3-(1,1,2,2-tetrafluoroethoxy)-phenyl]-pyridine-3,5-dicarboxylate,
3-methyl 5-[2-(4,4-diphenylpiperid-1-yl)-ethyl] 1,4-dihydro-2,6-dimethyl-4-[3-(1,1,2,2-tetrafluoroethoxy)-phenyl]-pyridine-3,5-dicarboxylate,
3-(2-methoxyethyl) 5-[2-(4,4-diphenylpiperid-1-yl)-ethyl] 1,4-dihydro-2,6-dimethyl-4-(3-nitrophenyl)-pyridine-3,5-dicarboxylate,
3-(2-methoxyethyl) 5-[3-(4,4-diphenylpiperid-1-yl]-propyl] 1,4-dihydro-2,6-dimethyl-4-(3-nitrophenyl)-pyridine-3,5-dicarboxylate,
3-methyl 5-{3-[4,4-di-(4-methoxyphenyl)-piperid-1-yl]-propyl} 1,4-dihydro-2,6-dimethyl-4-(3-nitrophenyl)-pyridine-3,5-dicarboxylate,
3-methyl 5-[4-(4,4-diphenylpiperid-1-yl)-butyl] 1,4-dihydro-2,6-dimethyl-4-(3-nitrophenyl)-pyridine-3,5-dicarboxylate,
3-methyl 5-[1,1-dimethyl-2-(4,4-diphenylpiperid-1-yl)-ethyl] 1,4-dihydro-2,6-dimethyl-4-(3-nitrophenyl)-pyridine-3,5-dicarboxylate,
3-ethyl 5-[3-(4,4-diphenylpiperid-1-yl)-propyl] 1,4-dihydro-2,6-dimethyl-4-(2-difluoromethoxyphenyl)-pyridine-3,5-dicarboxylate,
1,4-dihydro-2,6-dimethyl-4-(3-nitrophenyl)-pyridine-3,5-dicarboxylic acid 3-[3-(4,4-diphenylpiperid-1-yl)-propyl ester]
3-ethyl 5-[3-(4,4-diphenylpiperid-1-yl)-propyl] 1,4-dihydro-2,6-dimethyl-4-[3-(1,1,2,2-tetrafluoroethoxy)-phenyl]-pyridine-3,5-dicarboxylate,
3-ethyl 5-[3-(4,4-diphenylpiperid-1-yl)-propyl] 1,4-dihydro-2,6-dimethyl-4-(3-difluoromethoxyphenyl)-pyridine-3,5-dicarboxylate,
3-methyl 5-[3-(4,4-diphenylpiperid-1-yl)-propyl] 4-(2,3-dichlorophenyl)-1,4-dihydro-2,6-dimethylpyridine-3,5-dicarboxylate.
3-methyl 5-[3-(4,4-diphenylpiperid-1-yl)-propyl] 4-(2,1,3-benzoxdiazol-4-yl)-1,4-dihydro-2,6-dimethylpyridine-3,5-dicarboxylate,
3-methyl 5-[3-(4,4-diphenylpiperid-1-yl)-propyl] 1,4-dihydro-2,6-dimethyl-4-(3-fluorophenyl)-pyridine-3,5-dicarboxylate,
3-methyl 5-[3-(4,4-diphenylpiperid-1-yl)-propyl] 1,4-dihydro-2,6-dimethyl-4-(2-trifluoromethylphenyl)-pyridine-3,5-dicarboxylate,
3-ethyl 5-[3-(4,4-diphenylpiperid-1-yl)-propyl] 4-(2-cyanophenyl)-1,4-dihydro-2,6-dimethylpyridine-3,5-dicarboxylate and
3-methyl 5-[3-(4,4-diphenylpiperid-1-yl)-propyl] 4-(2-chlorophenyl)-1,4-dihydro-2,6-dimethylpyridine-3,5-dicarboxylatehydrochloride
and their salts.

**12.** 3-Methyl 5-[3-(4,4-diphenylpiperid-1-yl)-propyl] 1,4-dihydro-2,6-dimethyl-4-(3-nitrophenyl)-pyridine-3,5-dicarboxylate and its salts.

**13.** Process for the preparation of compounds of the formula I according to Claim 1 and their salts, characterised in that

a) cinnamic acid derivatives of the formula II

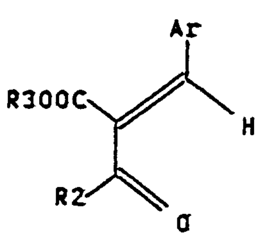

(II)

are reacted with enamine derivatives of the formula III

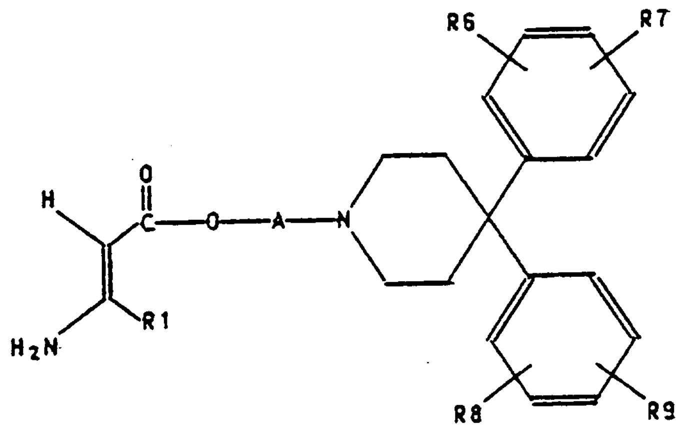

(III)

or

b) cinnamic acid derivatives of the formula II are reacted with ammonia and $\beta$-ketocarboxylic acid derivatives of the formula IV

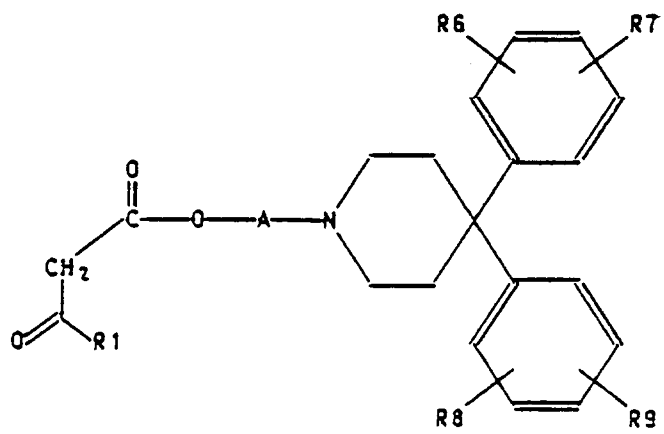

(IV)

or

c) enamines of the formula V

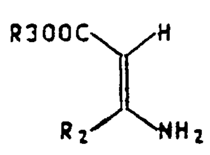

(V)

are reacted with benzylidenecarboxylic acid derivatives of the formula VI

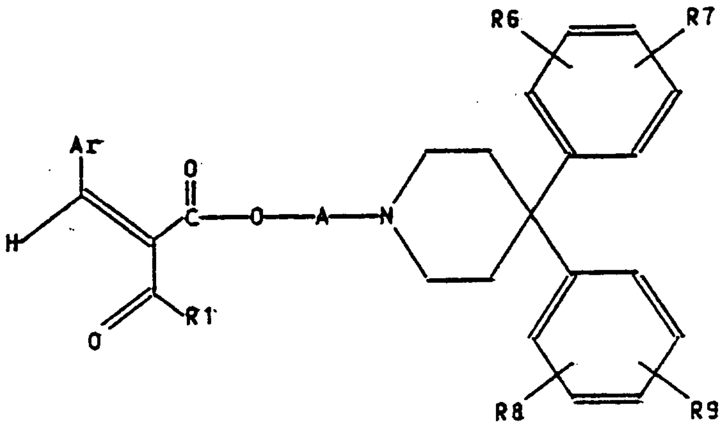

(VI)

or

d) keto compounds of the formula VII

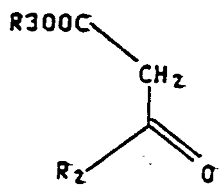

(VII)

are reacted with ammonia and benzylidenecarboxylic acid derivatives of the formula VI, or

e) aldehydes of the formula VIII

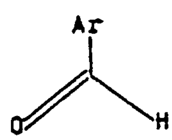

(VIII)

are reacted with enamines of the formula V and $\beta$-ketocarboxylic acid derivatives of the formula IV, or

f) aldehydes of the formula VIII are reacted with enamine derivatives of the formula III and keto compounds of the formula VII, or

g) 1,4-dihydropyridines of the formula IX

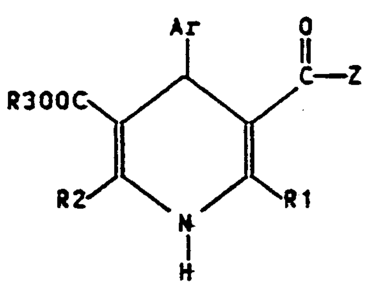

(IX)

are reacted with diaryl compounds of the formula X

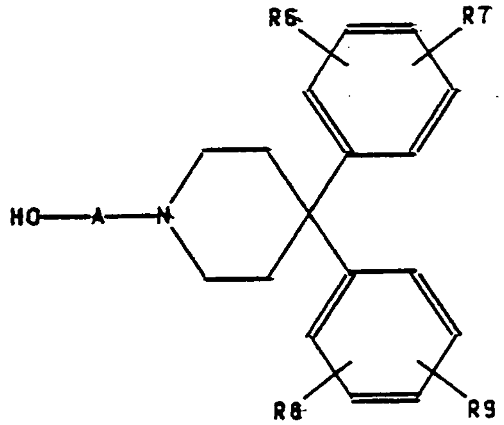

(X)

as such or in the form of their salts, and, if desired, resulting salts are then converted into the free bases or resulting bases are converted into the salts, wherein Ar, R1, R2, R3, R4, R5, R6, R7, R8, R9, Y and A have the meanings given in Claim 1 and Z, together with the carbonyl group to which it is bonded, represents a carboxyl group or a reactive carboxylic acid derivative.

**14.** Medicaments containing one or more compounds according to one or more of Claims 1 to 12 and/or their pharmacologically acceptable salts.

**15.** Compounds according to one or more of Claims 1 to 12 and their pharmacologically acceptable salts for use in the treatment and/or prophylaxis of hypertension, coronary heart diseases, disturbances in peripheral and cerebral circulation and/or diseases based on an increased retention of water or sodium.

**16.** Use of compounds according to one or more of Claims 1 to 12 and their pharmacologically acceptable salts for the manufacture of medicaments for the treatment and/or prophylaxis of hypertension, coronary heart diseases, disturbances in peripheral and cerebral circulation and/or diseases based on an increased retention of water or sodium.

**Claims for the following Contracting State : AT**

**1.** Process for the preparation of compounds of the general formula I,

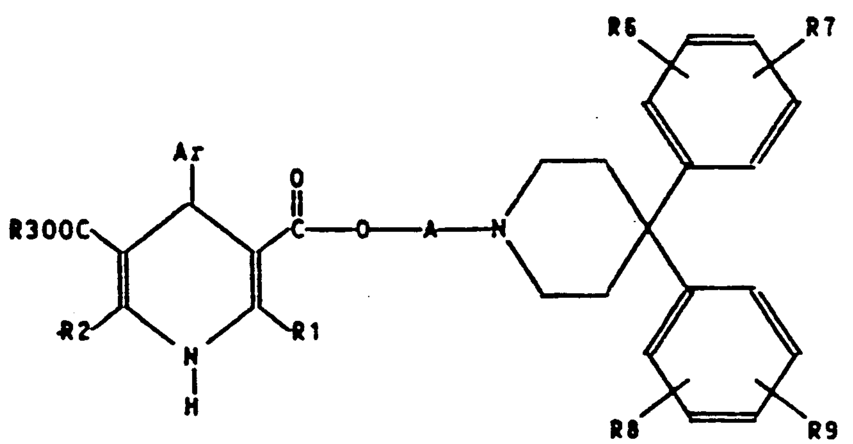

(I)

wherein Ar represents a ring of the formula

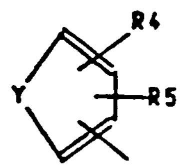

in which Y denotes oxygen (O), sulphur (S), vinylene (-CH = CH-), azomethine (-CH = N-) or a group of the formula

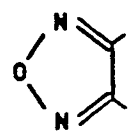

or

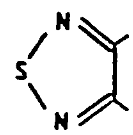

R1, R2 and R3 are identical or different and denote hydrogen, $C_1$-$C_6$-alkyl or $C_3$-$C_7$-alkoxyalkyl,
R4 and R5 are identical or different and denote hydrogen, hydroxyl, halogen, nitro, cyano, trifluoromethyl, $C_1$-$C_4$-alkyl, $C_1$-$C_4$-alkoxy, $C_1$-$C_4$-alkoxy which is completely or partly substituted by fluorine, $C_1$-$C_4$-alkoxycarbonyl, $C_2$-$C_5$-acyl, amino or monk- or di-$C_1$-$C_4$-alkylamino,
R6, R7, R8 and R9 are identical or different and denote hydrogen, hydroxyl, halogen, $C_1$-$C_4$-alkyl, $C_1$-$C_4$-alkoxy, amino, mono- or di-$C_1$-$C_4$-alkylamino, or $C_1$-$C_4$-alkoxy which is completely or partly substituted by fluorine, and
A denotes straight-chain or branched $C_2$-$C_5$-alkylene, and their salts, characterised in that

a) cinnamic acid derivatives of the formula II,

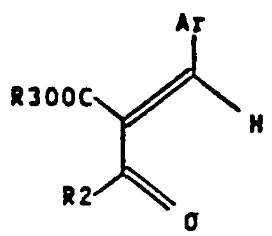

(II)

are reacted with enamine derivatives of the formula III

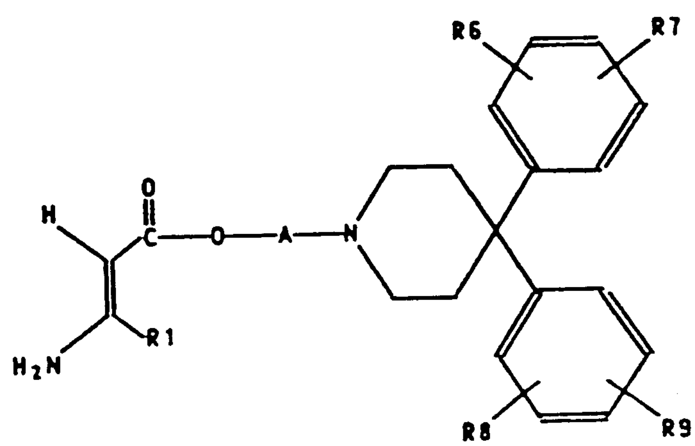

(III)

or

b) cinnamic acid derivatives of the formula II are reacted with ammonia and β-ketocarboxylic acid derivatives of the formula IV

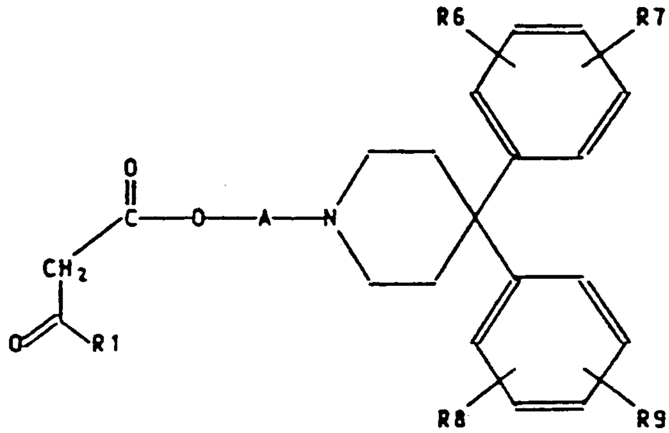

(IV)

or

c) enamines of the formula V

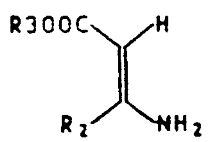

(V)

are reacted with benzylidenecarboxylic acid derivatives of the formula VI

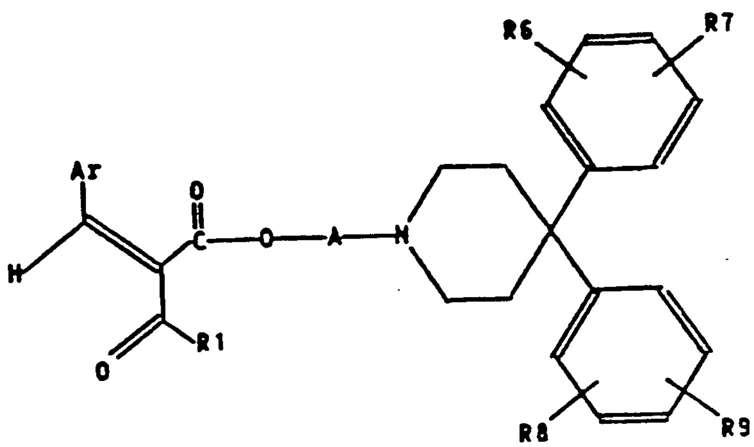

(VI)

or

d) keto compounds of the formula VII

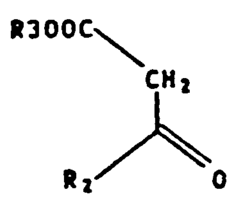

(VII)

are reacted with ammonia and benzylidenecarboxylic acid derivatives of the formula VI, or

e) aldehydes of the formula VIII

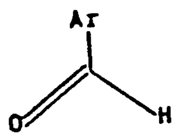

(VIII)

are reacted with enamines of the formula V and β-ketocarboxylic acid derivatives of the formula IV, or

f) aldehydes of the formula VIII are reacted with enamine derivatives of the formula III and keto compounds of the formula VII, or

g) 1,4-dihydropyridines of the formula IX

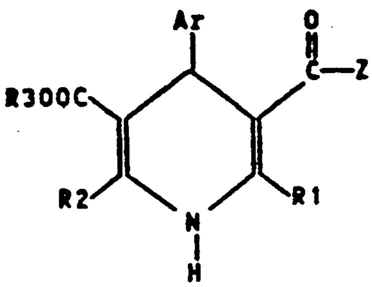

(IX)

are reacted with diaryl compounds of the formula X

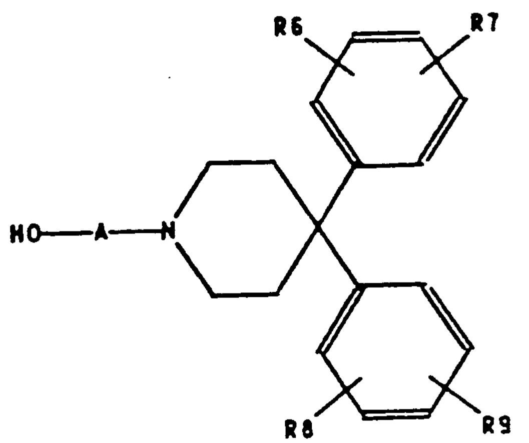

(X)

as such or in the form of their salts, and, if desired, resulting salts are then converted into the free bases or resulting bases are converted into the salts,

wherein Ar, R1, R2, R3, R4, R5, R6, R7, R8, R9, Y and A have the meanings given above and Z, together with the carbonyl group to which it is bonded, represents a carboxyl group or a reactive carboxylic acid derivative.

**2.** Process according to Claim 1, wherein Ar represents the ring mentioned in Claim 1, in which Y denotes vinylene (-CH = CH-), and R1, R2, R3, R4, R5, R6, R7, R8, R9 and A have the meanings given in Claim 1.

3. Process according to Claim 1, wherein Ar represents the ring mentioned in Claim 1, in which Y denotes

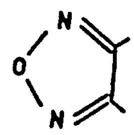

R4 and R5 denote hydrogen and R1, R2, R3, R6, R7, R8, R9 and A have the meanings given in Claim 1.

4. Process according to one of Claims 1 to 3, wherein R1 denotes $C_1$-$C_4$-alkyl, R2 denotes $C_1$-$C_4$-alkyl, R3 denotes $C_1$-$C_4$-alkyl or $C_3$-$C_5$-alkoxyalkyl, R4 denotes hydrogen, chlorine, nitro, methyl or methoxy, R5 denotes hydrogen, chlorine, fluorine, nitro, cyano, methyl, methoxy, difluoromethoxy, 1,1,2,2-tetrafluoroethoxy, trifluoromethyl or acetyl, R6 denotes hydrogen or methoxy, R7 denotes hydrogen, hydroxyl, chlorine, methyl, methoxy, difluoromethoxy or 1,1,2,2-tetrafluoroethoxy, R8 denotes hydrogen or methoxy, R9 denotes hydrogen, hydroxyl, chlorine, methyl, methoxy, difluoromethoxy or 1,1,2,2-tetrafluoroethoxy and A denotes ethylene or propylene.

5. Process according to one of Claims 1 to 3, wherein R1 denotes methyl, R2 denotes methyl, R3 denotes methyl, ethyl or methoxyethyl, R4 denotes hydrogen, R5 denotes hydrogen, chlorine, fluorine, nitro, cyano, methyl, methoxy, difluoromethoxy, 1,1,2,2-tetrafluoroethoxy or trifluoromethyl, R6 denotes hydrogen, R7 denotes hydrogen, hydroxyl, chlorine, methyl, methoxy, difluoromethoxy or 1,1,2,2-tetrafluoroethoxy, R8 denotes hydrogen, R9 denotes hydrogen, hydroxyl, chlorine, methyl, methoxy, difluoromethoxy or 1,1,2,2-tetrafluoroethoxy and A denotes ethylene or propylene.

6. Process according to Claim 2, wherein R1 denotes methyl, R2 denotes methyl, R3 denotes methyl, ethyl or methoxyethyl, R4 denotes hydrogen or chlorine, R5 denotes hydrogen, chlorine, fluorine, nitro, cyano, difluoromethoxy, 1,1,2,2-tetrafluoroethoxy or trifluoromethyl, R6 denotes hydrogen, R7 denotes hydrogen or methoxy, R8 denotes hydrogen, R9 denotes hydrogen or methoxy and A denotes ethylene, propylene, butylene, 1,1-dimethylethylene or 2,2-dimethylethylene.

7. Process according to Claim 1, wherein Ar denotes phenyl, 2-nitrophenyl, 3-nitrophenyl, 2-cyanophenyl, 3-cyanophenyl, 2-(1,1,2,2-tetrafluoroethoxy)-phenyl, 3-(1,1,2,2-tetrafluoroethoxy)-phenyl, 2-difluoromethoxyphenyl, 3-difluoromethoxyphenyl, 2-chlorophenyl, 3-chlorophenyl, 2,3-dichlorophenyl, 2-fluorophenyl, 3-fluorophenyl, 2-trifluoromethylphenyl or 3-trifluoromethylphenyl, R1 denotes methyl, R2 denotes methyl, R3 denotes methyl, ethyl or methoxyethyl, R6 denotes hydrogen, R7 denotes hydrogen or methoxy, R8 denotes hydrogen, R9 denotes hydrogen or methoxy and A denotes ethylene, propylene, butylene, 1,1-dimethylethylene or 2,2-dimethylethylene.

8. Process according to Claim 3, wherein R1 denotes methyl, R2 denotes methyl, R3 denotes methyl, ethyl or methoxyethyl, R4 denotes hydrogen, R5 denotes hydrogen, R6 denotes hydrogen, R7 denotes hydrogen or methoxy, R8 denotes hydrogen, R9 denotes hydrogen or methoxy and A denotes ethylene, propylene, butylene, 1,1-dimethylethylene or 2,2-dimethylethylene.

9. Process according to Claim 1, wherein Ar denotes 2-nitrophenyl or 3-nitrophenyl, R1, R2 and R3 are identical or different and denote $C_1$-$C_6$-alkyl or $C_3$-$C_7$-alkoxyalkyl, A represents straight-chain or branched $C_2$-$C_5$-alkylene and R6, R7, R8 and R9 denote hydrogen .

10. Process according to Claim 1, wherein Ar denotes 2-nitrophenyl or 3-nitrophenyl, R1 denotes methyl, R2 denotes methyl, R3 denotes methyl, ethyl or methoxyethyl, R6 denotes hydrogen, R7 denotes hydrogen, R8 denotes hydrogen, R9 denotes hydrogen and A denotes ethylene or propylene.

11. Process according to claim 1, characterised in that a compound selected from the group consisting of
3-methyl 5-[3-(4,4-diphenylpiperid-1-yl)-propyl] 1,4-dihydro-2,6-dimethyl-4-(3-nitrophenyl)-pyridine-3,5-dicarboxylate,
3-methyl 5-(2-(4,4-diphenylpiperid-1-yl)-ethyl] 1,4-dihydro-2,6-dimethyl-4-(3-nitrophenyl)-pyridine-3,5-dicarboxylate,

3-methyl 5-[3-(4,4-diphenylpiperid-1-yl)-propyl] 4-(3-cyanophenyl)-1,4-dihydro-2,6-dimethylpyridine-3,5-dicarboxylate,
3-methyl 5-[2-(4,4-diphenylpiperid-1-yl)-2-methyl-propyl] 1,4-dihydro-2,6-dimethyl-4-(3-nitrophenyl)-pyridine-3,5-dicarboxylate,
3-ethyl 5-[3-(4,4-diphenylpiperid-1-yl)-propyl] 1,4-dihydro-2,6-dimethyl-4-(3-nitrophenyl)-pyridine-3,5-dicarboxylate,
3-methyl 5-[3-(4,4-diphenylpiperid-1-yl)-propyl] 1,4-dihydro-2,6-dimethyl-4-[3-(1,1,2,2-tetrafluoroethoxy)-phenyl]-pyridine-3,5-dicarboxylate,
3-methyl 5-[2-(4,4-diphenylpiperid-1-yl)-ethyl] 1,4-dihydro-2,6-dimethyl-4-[3-(1,1,2,2-tetrafluoroethoxy)-phenyl]-pyridine-3,5-dicarboxylate,
3-(2-methoxyethyl) 5-[2-(4,4-diphenylpiperid-1-yl)-ethyl] 1,4-dihydro-2,6-dimethyl-4-(3-nitrophenyl)-pyridine-3,5-dicarboxylate,
3-(2-methoxyethyl) 5-[3-(4,4-diphenylpiperid-1-yl)-propyl] 1,4-dihydro-2,6-dimethyl-4-(3-nitrophenyl)-pyridine-3,5-dicarboxylate,
3-methyl 5-{3-[4,4-di-(4-methoxyphenyl)-piperid-1-yl]-propyl} 1,4-dihydro-2,6-dimethyl-4-(3-nitrophenyl)-pyridine-3,5-dicarboxylate,
3-methyl 5-[4-(4,4-diphenylpiperid-1-yl)-butyl] 1,4-dihydro-2,6-dimethyl-4-(3-nitrophenyl)-pyridine-3,5-dicarboxylate,
3-methyl 5-[1,1-dimethyl-2-(4,4-diphenylpiperid-1-yl)-ethyl] 1,4-dihydro-2,6-dimethyl-4-(3-nitrophenyl)-pyridine-3,5-dicarboxylate,
3-ethyl 5-[3-(4,4-diphenylpiperid-1-yl)-propyl] 1,4-dihydro-2,6-dimethyl-4-(2-difluoromethoxyphenyl)-pyridine-3,5-dicarboxylate,
1,4-dihydro-2,6-dimethyl-4-(3-nitrophenyl)-pyridine-3,5-dicarboxylic acid 3-[3-(4,4-diphenylpiperid-1-yl)-propyl ester]
3-ethyl 5-[3-(4,4-diphenylpiperid-1-yl)-propyl] 1,4-dihydro-2,6-dimethyl-4-[3-(1,1,2,2-tetrafluoroethoxy)-phenyl]-pyridine-3,5-dicarboxylate,
3-ethyl 5-[3-(4,4-diphenylpiperid-1-yl)-propyl] 1,4-dihydro-2,6-dimethyl-4-(3-difluoromethoxyphenyl)-pyridine-3,5-dicarboxylate,
3-methyl 5-[3-(4,4-diphenylpiperid-1-yl)-propyl] 4-(2,3-dichlorophenyl)-1,4-dihydro-2,6-dimethylpyridine-3,5-dicarboxylate,
3-methyl 5-[3-(4,4-diphenylpiperid-1-yl)-propyl] 4-(2,1,3-benzoxdiazol-4-yl]-1,4-dihydro-2,6-dimethylpyridine-3,5-dicarboxylate,
3-methyl 5-[3-(4,4-diphenylpiperid-1-yl)-propyl] 1,4-dihydro-2,6-dimethyl-4-(3-fluorophenyl)-pyridine-3,5-dicarboxylate,
3-methyl 5-[3-(4,4-diphenylpiperid-1-yl)-propyl] 1,4-dihydro-2,6-dimethyl-4-(2-trifluoromethylphenyl)-pyridine-3,5-dicarboxylate,
3-ethyl 5-[3-(4,4-diphenylpiperid-1-yl)-propyl] 4-(2-cyanophenyl)-1,4-dihydro-2,6-dimethylpyridine-3,5-dicarboxylate and
3-methyl 5-[3-(4,4-diphenylpiperid-1-yl)-propyl] 4-(2-chlorophenyl)-1,4-dihydro-2,6-dimethylpyridine-3,5-dicarboxylatehydrochloride, and their salts is prepared.

**12.** Process according to claim 1, characterised in that 3-methyl 5-[3-(4,4-diphenylpiperid-1-yl)-propyl] 1,4-dihydro-2,6-dimethyl-4-(3-nitrophenyl)-pyridine-3,5-dicarboxylate, or a salt thereof is prepared.

**13.** Process for the preparation of medicaments, characterised in that one or more compounds of formula I, prepared according to the process of claim 1, and/or their pharmaceutically acceptable salts are mixed with a pharmaceutical auxiliary and/or carrier.

**14.** Use of compounds of formula I and/or their pharmacologically acceptable salts for the manufacture of medicaments for the treatment and/or prophylaxis of hypertension, coronary heart diseases, disturbances in peripheral and cerebral circulation and/or diseases based on an increased retention of water or sodium.

## Revendications

**Revendications pour les Etats contractants suivants : BE, CH, DE, FR, GB, IT, LI, LU, NL, SE**

**1.** Composés de la formule générale I

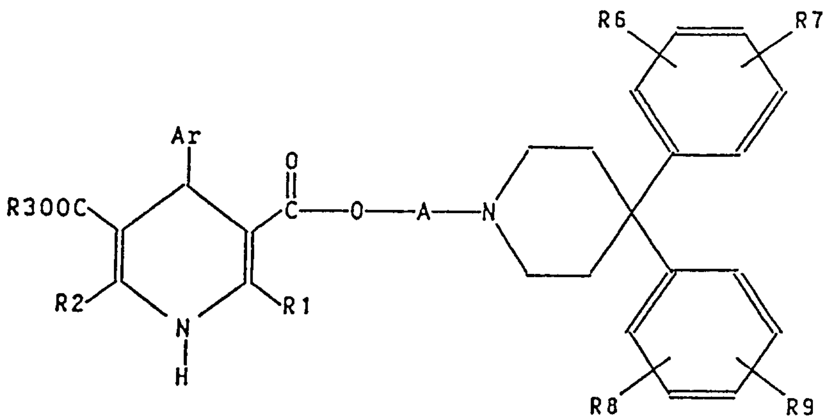

(I),

où Ar représente un cycle de la formule

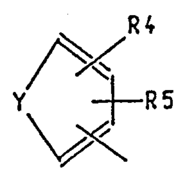

dans lequel Y désigne un oxygène (O), un soufre (S), un groupement vinylène (-CH = CH-), azométhine (-CH = N-), ou un groupement de la formule

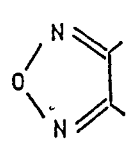

ou

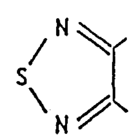

R1, R2 et R3 sont identiques ou différents et contiennent un hydrogène, un groupement $C_1$-$C_6$-alcoyle ou $C_3$-$C_7$-alcoxyalcoyle,
R4 et R5 sont identiques ou différents et contiennent un hydrogène, un radical hydroxy, un halogène, un groupement nitro, cyano, trifluorométhyle, $C_1$-$C_4$-alcoyle, $C_1$-$C_4$-alcoxy, un groupement $C_1$-$C_4$-alcoxy, substitué totalement ou en partie par du fluor, un radical $C_1$-C4-alcoxycarbonyle, $C_2$-$C_5$-acyle, amine, ou mono- ou di-$C_1$-$C_4$-alcoylamine,
R6, R7, R8 et R9 sont identiques ou différents et contiennent un hydrogène, un groupement hydroxy, un halogène, un radical $C_1$-$C_4$-alcoyle, $C_1$-$C_4$-alcoxy, amine, mono- ou di-$C_1$-$C_4$-alcoylamine, ou un groupement $C_1$-$C_4$-alcoxy, substitué totalement ou en partie par du fluor,
A désigne un radical $C_2$-$C_5$-alcoylène à chaîne linéaire ou ramifiée,
et leurs sels.

**2.** Composés de la formule I selon la revendication 1, caractérisés en ce que Ar représente le cycle représenté dans la revendication 1, dans lequel Y désigne un radical vinylène (-CH = CH-), et R1, R2, R3, R4, R5, R6, R7, R8, R9 et A ont la désignation donnée dans la revendication 1, et leurs sels.

**3.** Composés de la formule I selon la revendication 1, caractérisés en ce que Ar représente le cycle indiqué dans la revendication 1, où Y désigne

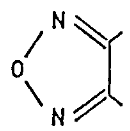

R4 et R5 un hydrogène, et ou R1, R2, R3, R6, R7, R8, R9 et A ont la signification donnée dans la revendication 1, et leurs sels.

4. Composés selon l'une des revendications 1 à 3, caractérisés en ce que R1 désigne un radical $C_1$-$C_4$-alcoyle, R2, un radical $C_1$-$C_4$-alcoyle, R3, un radical $C_1$-$C_4$-alcoyle ou $C_3$-$C_5$-alcoxyalcoyle, R4, un hydrogène, un chlore, un radical nitro, méthyle ou méthoxy, R5, un hydrogène, un chlore, un fluor, un radical nitro, cyano, méthyle, méthoxy, difluorométhoxy, 1,1,2,2-tétrafluoréthoxy, trifluorométhyle ou acétyle, R6, un hydrogène ou un radical méthoxy, R7, un hydrogène, un radical hydroxy, un chlore, un groupement méthyle, méthoxy, difluorométhoxy ou 1,1,2,2-tétrafluoréthoxy, R8, un hydrogène ou un radical méthoxy, R9, un hydrogène, un groupement hydroxy, un chlore, un radical méthyle, méthoxy, difluorométhoxy, ou 1,1,2,2-tétrafluoréthoxy, et A, un groupement éthylène ou propylène, et leurs sels.

5. Composés selon l'une des revendications 1 à 3, caractérisés en ce que R1 désigne un radical méthyle, R2, un radical méthyle, R3, un radical méthyle, éthyle, ou méthoxyéthyle, R4, un hydrogène, R5, un hydrogène, un chlore, un fluor, un groupement nitro, cyano, méthyle, méthoxy, difluorométhoxy, 1,1,2,2-tétrafluoréthoxy, ou trifluorométhyle, R6, un hydrogène, R7, un hydrogène, un groupement hydroxy, un chlore, un radical méthyle, méthoxy, difluorométhoxy ou 1,1,2,2-tétrafluoréthoxy, R8, un hydrogène, R9, un hydrogène, un groupement hydroxy, un chlore, un radical méthyle, méthoxy, difluorométhoxy, ou 1,1,2,2-tétrafluoréthoxy, et A, un radical éthylène ou propylène, et leurs sels.

6. Composés selon la revendication 2, caractérisés en ce que R1 désigne un radical méthyle, R2, un radical méthyle, R3, un radical méthyle, éthyle ou méthoxyéthyle, R4, un hydrogène ou un chlore, R5, un hydrogène, un chlore, un fluor, un groupement nitro, cyano, difluorométhoxy, 1,1,2,2-tétrafluoréthoxy ou trifluorométhyle, R6, un hydrogène, R7, un hydrogène ou un radical méthoxy, R8, un hydrogène, R9, un hydrogène ou un radical méthoxy, et A, un radical éthylène, propylène, butylène, 1,1-diméthyléthylène ou 2,2-diméthyléthylène, et leurs sels.

7. Composés selon la revendication 1, caractérisés en ce que Ar est un radical phényle, 2-nitrophényle, 3-nitrophényle, 2-cyanophényle, 3-cyanophényle, 2-(1,1,2,2-tétrafluoréthoxy)-phényle, 3-(1,1,2,2-tétrafluoréthoxy)-phényle, 2-difluorométhoxyphényle, 3-difluorométhoxyphényle, 2-chlorophényle, 3-chlorophényle, 2,3-dichlorophényle, 2-fluorophényle, 3-fluorophényle, 2-trifluorométhylphényle, ou 3-trifluorométhylphényle, R1, un radical méthyle, R2, un radical méthyle, R3, un radical méthyle, éthyle, ou méthoxyéthyle, R6, un hydrogène, R7, un hydrogène ou un groupement méthoxy, R8, un hydrogène, R9, un hydrogène ou un radical méthoxy et A, un radical éthylène, propylène, butylène, 1,1-diméthyléthylène ou 2,2-diméthyléthylène, et leurs sels.

8. Composés selon la revendication 3, caractérisé en ce que R1 désigne un radical méthyle, R2, un radical méthyle, R3, un radical méthyle, éthyle, ou méthoxyéthyle, R4, un hydrogène, R5, un hydrogène, R6, un hydrogène, R7, un hydrogène ou un radical méthoxy, R8, un hydrogène, R9, un hydrogène ou un radical méthoxy, et A, un radical éthylène, propylène, butylène, 1,1-diméthyléthylène ou 2,2-diméthyléthylène, et leurs sels.

9. Composés selon la revendication 1, caractérisés en ce que Ar désigne un radical 2-nitrophényle ou 3-nitrophényle, R1, R2 et R3 sont identiques ou différents et désignent un groupement $C_1$-$C_6$-alcoyle, ou $C_3$-$C_7$-alcoxyalcoyle, A un radical $C_2$-$C_5$-alcoylène à chaîne linéaire ou ramifiée, et R6, R7, R8 et R9 un hydrogène, et leurs sels.

10. Composés selon la revendication 1, caractérisés en ce que Ar désigne un radical 2-nitrophényle, ou 3-nitrophényle, R1, un radical méthyle, R2, un radical méthyle, R3, un radical méthyle, éthyle, ou méthoxyéthyle, R6, un hydrogène, R7 un hydrogène, R8 un hydrogène, R9 un hydrogène, et A, un radical éthylène, propylène, et leurs sels.

**11.** Composé choisi dans le groupe composé de

1) l'1,4-dihydro - 2,6-diméthyl - 4-(3-nitrophényl) - pyridine - 3,5-dicarboxylique - 3-méthyl - 5-[3-(4,4-diphénylpipéridyl-1) - propyl] - ester,
2) l'1,4-dihydro - 2,6-diméthyl - 4-(3-nitrophényl) - pyridine - 3,5-dicarboxylique - 3-méthyl - 5-[2-(4,4-diphénylpipéridyl-1) - éthyl] - ester,
3) le 4-(3-cyanophényl) - 1,4-dihydro - 2,6-diméthylpyridine - 3,5-dicarboxylique - 3-méthyl - 5-[3-(4,4-diphénylpipéridyl-1) - propyl] - ester,
4) l'1,4-dihydro - 2,6-diméthyl - 4-(3-nitrophényl) - pyridine - 3,5-dicarboxylique - 3-méthyl - 5-[2-(4,4-diphénylpipéridyl-1) - 2-méthyl - propyl] - ester,
5) l'1,4-dihydro - 2-6-diméthyl - 4-(3-nitrophényl) - pyridine - 3,5-dicarboxylique - 3-éthyl - 5-[3-(4,4-diphénylpipéridyl-1) - propyl] - ester,
6) l'1,4-dihydro - 2,6-diméthyl - 4-[3-(1,1,2,2-tétrafluoréthoxy) - phényl] - pyridine - 3,5-dicarboxylique - 3-méthyl - 5-[3-(4,4-diphénylpipéridyl-1) - propyl] - ester,
7) l'1,4-dihydro - 2,6-diméthyl - 4-[3-(1,1,2,2-tétrafluoréthoxy) - phényl] - pyridine - 3,5-dicarboxylique -3-méthyl - 5-[2-(4,4-diphénylpipéridyl-1) - éthyl] - ester,
8) l'1,4-dihydro - 2,6-diméthyl - 4-[3-nitrophényl] - pyridine - 3,5-dicarboxylique - 3-(2-méthoxyéthyl) - 5-[2-4,4-diphénylpipéridyl-1) - éthyl] - ester,
9) l'1,4-dihydro - 2,6-diméthyl - 4-(3-nitrophényl) - pyridine - 3,5-dicarboxylique - 3-(2-méthoxyéthyl) - 5-[3-(4,4-diphénylpipéridyl-1) - propyl] - ester,
10) l'1,4-dihydro - 2,6-diméthyl - 4-(3-nitrophényl) - pyridine - 3,5-dicarboxylique - 3-méthyl) - 5-{3-[4,4-di - (4-méthoxyphényl) - pipéridyl-1] - propyl} - ester,
11) l'1,4-dihydro - 2,6-diméthyl - 4-(3-nitrophényl) - pyridlne - 3,5-dicarboxylique - 3-méthyl - 5-[4-(4,4-diphénylpipéridyl-1) - butyl] - ester,
12) l'1,4-dihydro - 2,6-diméthyl - 4-(3-nitrophényl) - pyridine - 3,5-dicarboxylique - 3-méthyl - 5-[1,1-diméthyl - 2-(4,4-diphénylpipéridyl-1) - éthyl] - ester,
13) l'1,4-dihydro - 2,6-diméthyl - 4-(2-difluorométhoxyphényl) - pyridine - 3,5-dicarboxylique - 3-éthyl -5-[3-(4,4-diphénylpipéridyl-1) - propyl] - ester,
14) l'1,4-dihydro - 2,6-diméthyl - 4-(3-nitrophényl) - pyridine - 3,5-dicarboxylique - 3-[3-(4,4-diphénylpipéridyl-1) - propyl] - ester,
15) l'1,4-dihydro - 2,6-diméthyl - 4-(3-(1,1,2,2-tétrafluoréthoxy) - phényl] - pyridine - 3,5-dicarboxylique -3-éthyl - 5-[3-(4,4-diphénylpipéridyl-1) - propyl] - ester,
16) l'1,4-dihydro - 2,6-diméthyl - 4-(3-difluorométhoxyphényl) - pyridine - 3,5-dicarboxylique - 3-éthyl -5-(3-(4,4-diphénylpipéridyl-1) - propyl] - ester,
17) le 4-(2,3-dichlorophényl) - 1,4-dihydro - 2,6-diméthylpyridine - 3,5-dicarboxylique - 3-méthyl - 5-[3-(4,4-diphénylpipéridyl-1) - propyl] - ester,
18) le 4-(2,1,3-benzoxdiazol-4-yl) -1,4-dihydro - 2,6-diméthyl - pyridine - 3,5-dicarboxylique - 3-méthyl - 5-[3-(4,4-diphénylpipéridyl-1) - propyl] - ester,
19) l'1,4-dihydro - 2,6-diméthyl - 4-(3-fluorophényl) - pyridine - 3,5-dicarboxylique - 3-méthyl - 5-[3-(4,4-diphénylpipéridyl-1) - propyl] - ester,
20) l'1,4-dihydro - 2,6-diméthyl - 4-(2-trifluorométhylphényl) - pyridine - 3,5-dicarboxylique - 3-méthyl -5-(3-(4,4-diphénylpipéridyl-1) - propyl] - ester,
21) le 4-(2-cyanophényl) - 1,4-dihydro - 2,6-diméthylpyridine - 3,5-dicarboxylique - 3-éthyl - 5-[3-(4,4-diphénylpipéridyl-1) - propyl] - ester,
22) le 4-(2-chlorophényl) - 1,4-dihydro - 2,6-diméthylpyridine - 3,5-dicarboxylique - 3-méthyl - 5-[3-(4,4-diphénylpipéridyl-1) - propyl] - ester - hydrochlorure, et leurs sels.

**12.** L'1,4-dihydro - 2,6-diméthyl - 4-(3-nitrophényl) - pyridine - 3,5-dicarboxylique - 3-méthyl - 5-[3-(4,4-diphénylpipéridyl-1) - propyl] - ester et ses sels.

**13.** Procédé de préparation de composés de la formule I selon la revendication 1, et leurs sels, caractérisé en ce qu'on substitue

a) les dérivés de l'acide cinnamique de formule II

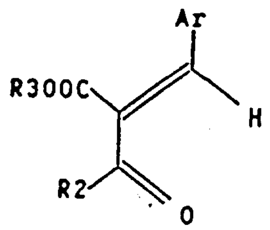

(II),

avec des dérivés d'énamines de la formule III

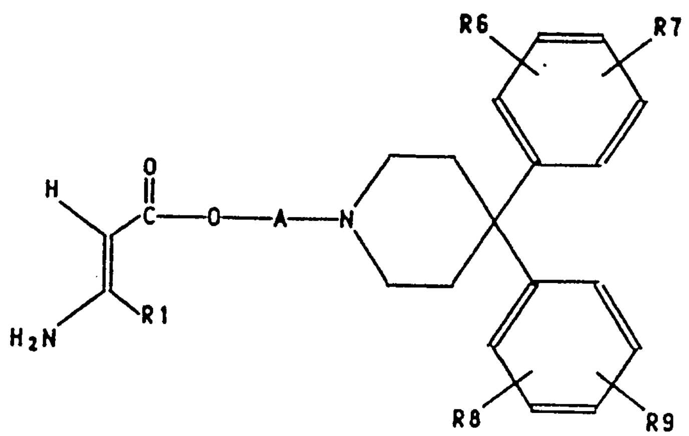

(III),

ou

b) des dérivés de l'acide cinnamique de la formule II avec de l'ammoniac et des dérivés de l'acide 8-cétocarboxylique de la formule IV

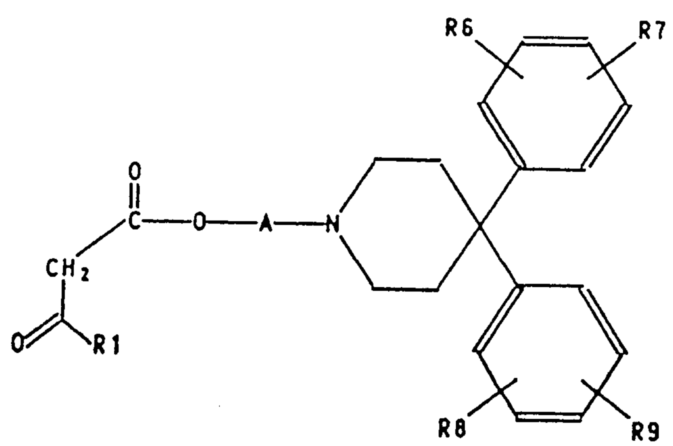

(IV),

ou

c) des énamines de la formule V

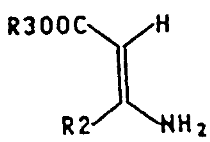

(V).

avec des dérivés de l'acide benzylidène-carboxylique de la formule VI

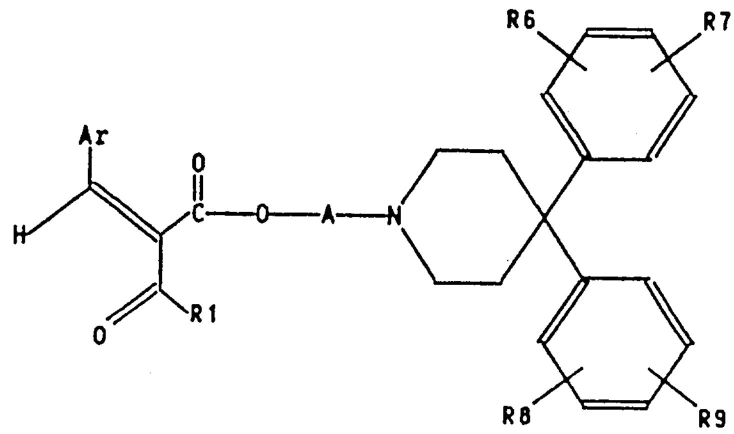

(VI),

ou

d) des composés cétoniques de la formule VII

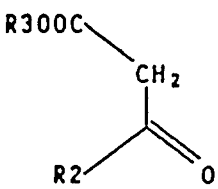

(VII),

avec de l'ammoniac et des dérivés de l'acide benzylidène-carboxylique de la formule VI, ou

e) des aldéhydes de la formule VIII

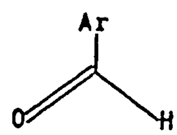

(VIII),

avec des énamines de la formule V et des dérivés de l'acide 8-cétocarboxylique de la formule IV, ou

f) des aldéhydes de la formule VIII avec des dérivés d'énamines de la formule III et des composés cétoniques de la formule VII, ou

g) des 1,4-dihydropyridines de la formule IX

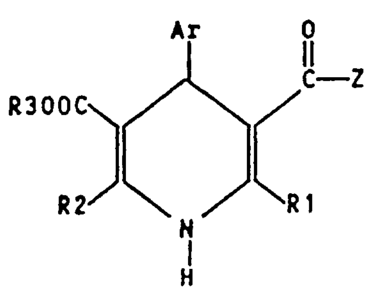

(IX),

avec des composés diaryles de la formule X

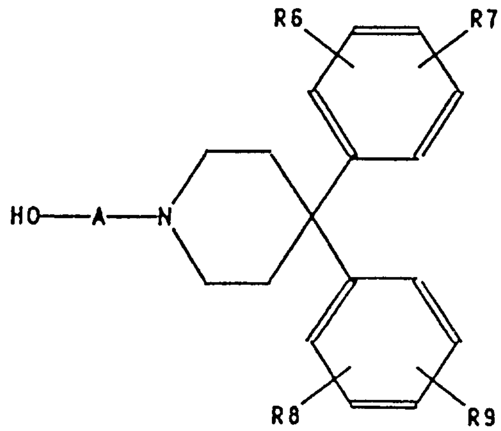

(X),

sous cette forme ou sous la forme de leurs sels, et qu'on transforme de préférence les sels ainsi obtenus en des bases libres ou les bases obtenues en sels, sachant que Ar, R1, R2, R3, R4, R5, R6, R7, R8, R9, Y et A désignent les radicaux énumérés à la revendication 1 et que Z représente conjointement avec le groupement carbonyle auquel il est lié, un groupement carboxylique ou un dérivé réactif de l'acide carboxylique.

**14.** Médicaments contenant un ou plusieurs composés selon une ou plusieurs des revendications 1 à 12 et/ou leurs sels pharmacologiquement supportés.

**15.** Composés selon une ou plusieurs des revendications 1 à 12 et leurs sels pharmacologiquement supportés pour une utilisation dans le traitement et/ou la prophylaxie de l'hypertonie, des cardiopathies coronariennes, des troubles circulatoires périphériques et cérébraux et/ou des maladies qui entraînent une rétention d'eau ou de sodium accrue.

**16.** Utilisation de composés selon une ou plusieurs des revendications 1 à 12 et de leurs sels pharmacologiquement supportés pour préparer des médicaments destinés au traitement et/ou à la prophylaxie de l'hypertonie, des cardiopathies coronariennes, des troubles circulatoires périphériques et cérébraux et/ou des maladies qui entraînent une rétention d'eau ou de sodium accrue.

**Revendications pour l'Etat contractant suivant : AT**

**1.** Procédé de préparation de composés de la formule générale I

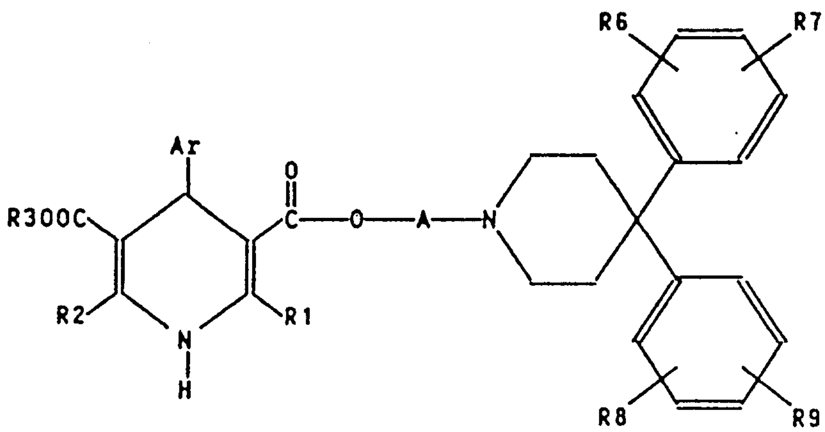

(I),

où Ar représente un cycle de la formule

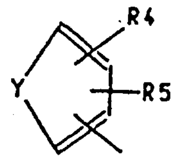

dans lequel Y désigne un oxygène (O), un soufre (S), un groupement vinylène (-CH = CH-), azométhine (-CH = N-), ou un groupement de la formule

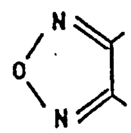

ou

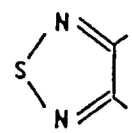

R1, R2 et R3 sont identiques ou différents et contiennent un hydrogène, un groupement $C_1$-$C_6$-alcoyle ou $C_3$-$C_7$-alcoxyalcoyle,
R4 et R5 sont identiques ou différents et contiennent un hydrogène, un radical hydroxy, un halogène, un groupement nitro, cyano, trifluorométhyle, $C_1$-$C_4$-alcoyle, $C_1$-$C_4$-alcoxy, un groupement $C_1$-$C_4$-alcoxy, substitué totalement ou en partie par du fluor, un radical $C_1$-C4-alcoxycarbonyle, $C_2$-$C_5$-acyle, amine, ou mono- ou di-$C_1$-$C_4$-alcoylamine,
R6, R7, R8 et R9 sont identiques ou différents et contiennent un hydrogène, un groupement hydroxy, un halogène, un radical $C_1$-$C_4$-alcoyle, $C_1$-$C_4$-alcoxy, amine, mono- ou di-$C_1$-$C_4$-alcoylamine, ou un groupement $C_1$-$C_4$-alcoxy, substitué totalement ou en partie par du fluor,
A désigne un radical $C_2$-$C_5$-alcoylène à chaîne linéaire ou ramifiée,
et leurs sels, caractérisé en ce qu'on substitue

a) les dérivés de l'acide cinnamique de formule II

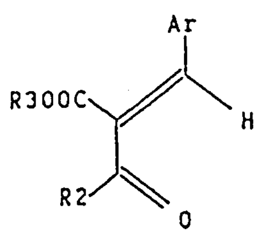

(II),

avec des dérivés d'énamines de la formule III

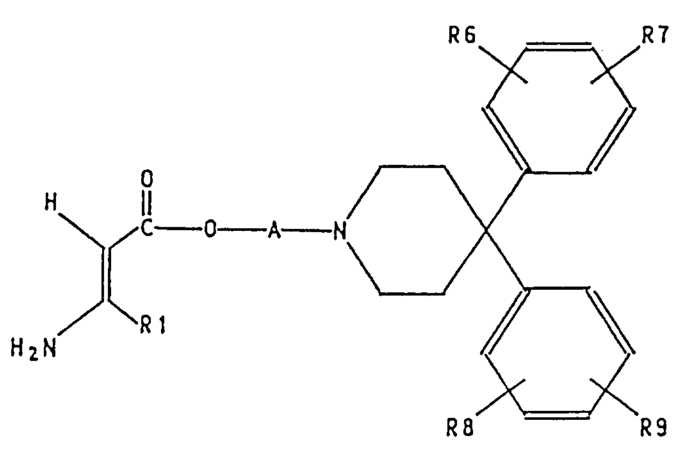

(III),

ou

b) des dérivés de l'acide cinnamique de la formule II avec de l'ammoniac et des dérivés de l'acide 8-cétocarboxylique de la formule IV

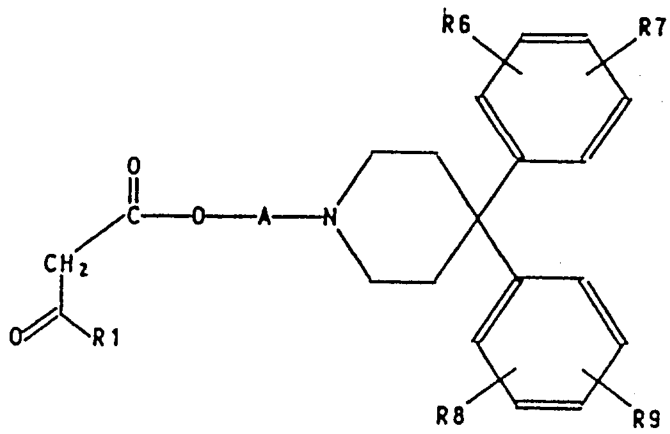

(IV),

ou

c) des énamines de la formule V

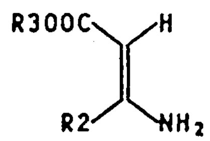

(V).

avec des dérivés de l'acide benzylidène-carboxylique de la formule VI

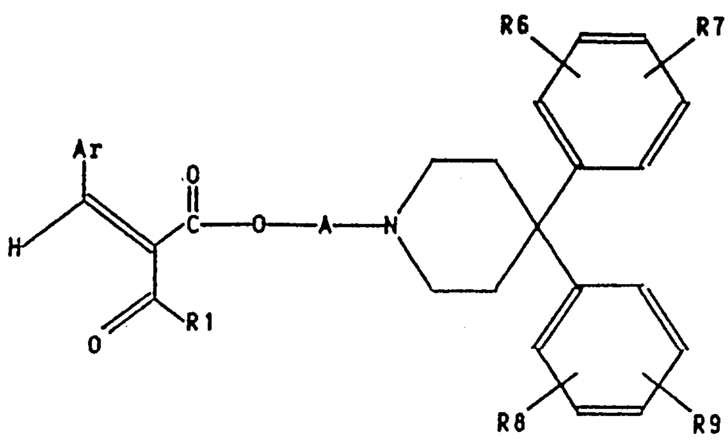

(VI),

ou

d) des composés cétoniques de la formule VII

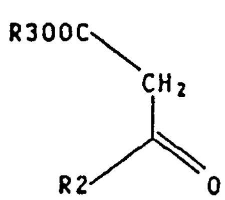

(VII),

avec de l'ammoniac et des dérivés de l'acide benzylidène-carboxylique de la formule VI, ou
e) des aldéhydes de la formule VIII

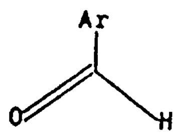

(VIII),

avec des énamines de la formule V et des dérivés de l'acide 8-cétocarboxylique de la formule IV, ou
f) des aldéhydes de la formule VIII avec des dérivés d'énamines de la formule III et des composés cétoniques de la formule VII, ou
g) des 1,4-dihydropyridines de la formule IX

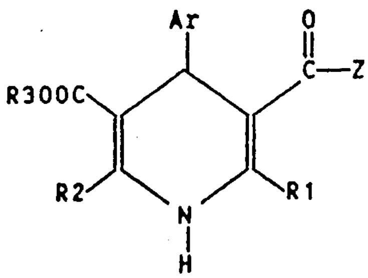

(IX),

avec des composés diaryles de la formule X

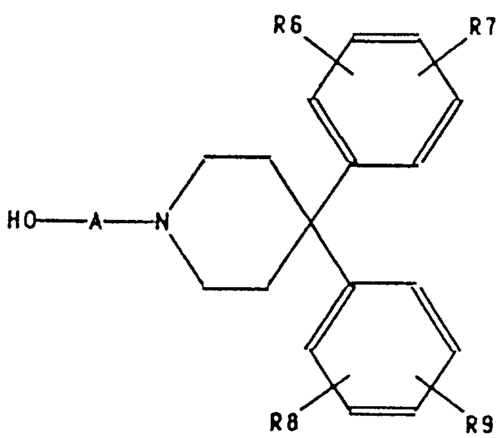

(X),

sous cette forme ou sous la forme de leurs sels, et qu'on transforme de préférence les sels ainsi obtenus en des bases libres ou les bases obtenues en sels, sachant que Ar, R1, R2, R3, R4, R5, R6, R7, R8, R9, Y et A désignent les radicaux énumérés plus haut et que Z représente conjointement avec le groupement carbonyle auquel il est lié, un groupement carboxylique ou un dérivé réactif de l'acide carboxylique.

2. Procédé selon la revendication 1, caractérisés en ce que Ar représente le cycle représenté dans la revendication 1, dans lequel Y désigne un radical vinylène (-CH=CH-), et R1, R2, R3, R4, R5, R6, R7, R8, R9 et A ont la désignation donnée dans la revendication 1.

3. Procédé selon la revendication 1, caractérises en ce que Ar représente le cycle indiqué dans la revendication 1, où Y désigne

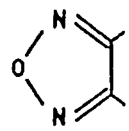

R4 et R5 un hydrogène, et où R1, R2, R3, R6, R7, R8, R9 et A ont la signification donnée dans la revendication 1.

**4.** Procédé selon l'une des revendications 1 à 3, caractérisé en ce que R1 désigne un radical $C_1$-$C_4$-alcoyle, R2, un radical $C_1$-$C_4$-alcoyle, R3, un radical $C_1$-$C_4$-alcoyle ou $C_3$-$C_5$-alcoxyalcoyle, R4, un hydrogène, un chlore, un radical nitro, méthyle ou méthoxy, R5, un hydrogène, un chlore, un fluor, un radical nitro, cyano, méthyle, méthoxy, difluorométhoxy, 1,1,2,2-tétrafluoréthoxy, trifluorométhyle ou acétyle, R6, un hydrogène ou un radical méthoxy, R7, un hydrogène, un radical hydroxy, un chlore, un groupement méthyle, méthoxy, difluorométhoxy ou 1,1,2,2-tétrafluoréthoxy, R8, un hydrogène ou un radical méthoxy, R9, un hydrogène, un groupement hydroxy, un chlore, un radical méthyle, méthoxy, difluorométhoxy, ou 1,1,2,2-tétrafluoréthoxy, et A, un groupement éthylène ou propylène.

**5.** Procédé selon l'une des revendications 1 à 3, caractérisé en ce que R1 désigne un radical méthyle, R2, un radical méthyle, R3, un radical méthyle, éthyle, ou méthoxyéthyle, R4, un hydrogène, R5, un hydrogène, un chlore, un fluor, un groupement nitro, cyano, méthyle, méthoxy, difluorométhoxy, 1,1,2,2-tétrafluoréthoxy, ou trifluorométhyle, R6, un hydrogène, R7, un hydrogène, un groupement hydroxy, un chlore, un radical méthyle, méthoxy, difluorométhoxy ou 1,1,2,2-tétrafluoréthoxy, R8, un hydrogène, R9, un hydrogène, un groupement hydroxy, un chlore, un radical méthyle, méthoxy, difluorométhoxy, ou 1,1,2,2-tétrafluoréthoxy, et A, un radical éthylène ou propylène.

**6.** Procédé selon la revendication 2, caractérisé en ce que R1 désigne un radical méthyle, R2, un radical méthyle, R3, un radical méthyle, éthyle ou méthoxyéthyle, R4, un hydrogène ou un chlore, R5, un hydrogène, un chlore, un fluor, un groupement nitro, cyano, difluorométhoxy, 1,1,2,2-tétrafluoréthoxy ou trifluorométhyle, R6, un hydrogène, R7, un hydrogène ou un radical méthoxy, R8, un hydrogène, R9, un hydrogène ou un radical méthoxy, et A, un radical éthylène, propylène, butylène, 1,1-diméthyléthylène ou 2,2-diméthyléthylène.

**7.** Procédé selon la revendication 1, caractérisé en ce que Ar est un radical phényle, 2-nitrophényle, 3-nitrophényle, 2-cyanophényle, 3-cyanophényle, 2-(1,1,2,2-tétrafluoréthoxy)-phényle, 3-(1,1,2,2-tétrafluoréthoxy)-phényle, 2-difluorométhoxyphényle, 3-difluorométhoxyphényle, 2-chlorophényle, 3-chlorophényle, 2,3-dichlorophényle, 2-fluorophényle, 3-fluorophényle, 2-trifluorométhylphényle, ou 3-trifluorométhylphényle, R1, un radical méthyle, R2, un radical méthyle, R3, un radical méthyle, éthyle, ou méthoxyéthyle, R6, un hydrogène, R7, un hydrogène ou un groupement méthoxy, R8, un hydrogène, R9, un hydrogène ou un radical méthoxy et A, un radical éthylène, propylène, butylène, 1,1-diméthyléthylène ou 2,2-diméthyléthylène.

**8.** Procédé selon la revendication 3, caractérisé en ce que R1 désigne un radical méthyle, R2, un radical méthyle, R3, un radical méthyle, éthyle, ou méthoxyéthyle, R4, un hydrogène, R5, un hydrogène, R6, un hydrogène, R7, un hydrogène ou un radical méthoxy, R8, un hydrogène, R9, un hydrogène ou un radical méthoxy, et A, un radical éthylène, propylène, butylène, 1,1-diméthyléthylène ou 2,2-diméthyléthylène.

**9.** Procédé selon la revendication 1, caractérisé en ce que Ar désigne un radical 2-nitrophényle ou 3-nitrophényle, R1, R2 et R3 sont identiques ou différents et désignent un groupement $C_1$-$C_6$-alcoyle, ou $C_3$-$C_7$-alcoxyalcoyle, A un radical $C_2$-$C_5$-alcoylène à chaîne linéaire ou ramifiée, et R6, R7, R8 et R9 un hydrogène.

**10.** Procédé selon la revendication 1, caractérisé en ce que Ar désigne un radical 2-nitrophényle, ou 3-nitrophényle, R1, un radical méthyle, R2, un radical méthyle, R3, un radical méthyle, éthyle, ou méthoxyéthyle, R6, un hydrogène, R7 un hydrogène, R8 un hydrogène, R9 un hydrogène, et A, un radical éthylène, propylène.

**11.** Procédé selon la revendication 1, caractérisé en ce qu'on prépare un composé choisi dans le groupe composé de

1) l'1,4-dihydro - 2,6-diméthyl - 4-(3-nitrophényl) - pyridine - 3,5-dicarboxylique - 3-méthyl - 5-[3-(4,4-diphénylpipéridyl-1) - propyl] - ester,
2) l'1,4-dihydro - 2,6-diméthyl - 4-(3-nitrophényl) - pyridine - 3,5-dicarboxylique - 3-méthyl - 5-[2-(4,4-diphénylpipéridyl-1) - éthyl] - ester,
3) le 4-(3-cyanophényl) - 1,4-dihydro - 2,6-diméthylpyridine - 3,5-dicarboxylique - 3-méthyl - 5-[3-(4,4-diphénylpipéridyl-1) - propyl] - ester,
4) l'1,4-dihydro - 2,6-diméthyl - 4-(3-nitrophényl) - pyridine - 3,5-dicarboxylique - 3-méthyl - 5-[2-(4,4-diphénylpipéridyl-1) - 2-méthyl - propyl] - ester,
5) l'1,4-dihydro - 2-6-diméthyl - 4-(3-nitrophényl) - pyridine - 3,5-dicarboxylique - 3-éthyl - 5-[3-(4,4-diphénylpipéridyl-1) - propyl] - ester,
6) l'1,4-dihydro - 2,6-diméthyl - 4-[3-(1,1,2,2-tétrafluoréthoxy) - phényl] - pyridine - 3,5-dicarboxylique - 3-méthyl - 5-[3-(4,4-diphénylpipéridyl-1) - propyl] - ester,
7) l'1,4-dihydro - 2,6-diméthyl - 4-[3-(1,1,2,2-tétrafluoréthoxy) - phényl] - pyridine - 3,5-dicarboxylique -3-méthyl - 5-[2-(4,4-diphénylpipéridyl-1) - éthyl] - ester,
8) l'1,4-dihydro - 2,6-diméthyl - 4-[3-nitrophényl] - pyridine - 3,5-dicarboxylique - 3-(2-méthoxyéthyl) - 5-[2-4,4-diphénylpipéridyl-1) - éthyl] - ester,
9) l'1,4-dihydro - 2,6-diméthyl - 4-(3-nitrophényl) - pyridine - 3,5-dicarboxylique - 3-(2-méthoxyéthyl) - 5-[3-(4,4-diphénylpipéridyl-1) - propyl] - ester,
10) l'1,4-dihydro - 2,6-diméthyl - 4-(3-nitrophényl) - pyridine - 3,5-dicarboxylique - 3-méthyl) - 5-{3-[4,4-di - (4-méthoxyphényl) - pipéridyl-1] - propyl} - ester,
11) l'1,4-dihydro - 2,6-diméthyl - 4-(3-nitrophényl) - pyridine - 3,5-dicarboxylique - 3-méthyl - 5-[4-(4,4-diphénylpipéridyl-1) - butyl] - ester,
12) l'1,4-dihydro - 2,6-diméthyl - 4-(3-nitrophényl) - pyridine - 3,5-dicarboxylique - 3-méthyl - 5-[1,1-diméthyl - 2-(4,4-diphénylpipéridyl-1) - éthyl] - ester,
13) l'1,4-dihydro - 2,6-diméthyl - 4-(2-difluorométhoxyphényl) - pyridine - 3,5-dicarboxylique - 3-éthyl -5-[3-(4,4-diphénylpipéridyl-1) - propyl] - ester,
14) l'1,4-dihydro - 2,6-diméthyl - 4-(3-nitrophényl) - pyridine - 3,5-dicarboxylique - 3-[3-(4,4-diphénylpipéridyl-1) - propyl] - ester,
15) l'1,4-dihydro - 2,6-diméthyl - 4-[3-(1,1,2,2-tétrafluoréthoxy) - phényl] - pyridine - 3,5-dicarboxylique -3-éthyl - 5-[3-(4,4-diphénylpipéridyl-1) - propyl] - ester,
16) l'1,4-dihydro - 2,6-diméthyl - 4-(3-difluorométhoxyphényl) - pyridine - 3,5-dicarboxylique - 3-éthyl -5-[3-(4,4-diphénylpipéridyl-1) - propyl] - ester,
17) le 4-(2,3-dichlorophényl) - 1,4-dihydro - 2,6-diméthylpyridine - 3,5-dicarboxylique - 3-méthyl - 5-[3-(4,4-diphénylpipéridyl-1) - propyl] - ester,
18) le 4-(2,1,3-benzoxdiazol-4-yl) -1,4-dihydro - 2,6-diméthyl - pyridine - 3,5-dicarboxylique - 3-méthyl - 5-[3-(4,4-diphénylpipéridyl-1) - propyl] - ester,
19) l'1,4-dihydro - 2,6-diméthyl - 4-(3-fluorophényl) - pyridine - 3,5-dicarboxylique - 3-méthyl - 5-[3-(4,4-diphénylpipéridyl-1) - propyl] - ester,
20) l'1,4-dihydro - 2,6-diméthyl - 4-(2-trifluorométhylphényl) - pyridine - 3,5-dicarboxylique - 3-méthyl -5-[3-(4,4-diphénylpipéridyl-1) - propyl] - ester,
21) le 4-(2-cyanophényl) - 1,4-dihydro - 2,6-diméthylpyridine - 3,5-dicarboxylique - 3-éthyl - 5-[3-(4,4-diphénylpipéridyl-1) - propyl] - ester,
22) le 4-(2-chlorophényl) - 1,4-dihydro - 2,6-diméthylpyridine - 3,5-dicarboxylique - 3-méthyl - 5-[3-(4,4-diphénylpipéridyl-1) - propyl] - ester - hydrochlorure, et leurs sels.

**12.** Procédé selon la revendication 1, caractérisé en ce qu'on prépare l'1,4-dihydro - 2,6-diméthyl - 4-(3-nitrophényl) - pyridine - 3,5-dicarboxylique - 3-méthyl - 5-[3-(4,4-diphénylpipéridyl-1) - propyl] - ester ou un sel qui en dérive.

**13.** Procédé de préparation de médicaments, caractérisé en ce qu'on mélange un ou plusieurs composés de la formule I, préparés selon le procédé de la revendication 1, et/ou leurs sels pharmacologiquement supportés, et un support et/ou un agent auxiliaire pharmaceutiques.

**14.** Utilisation de composés de la formule I et/ou de leurs sels pharmacologlquement supportés, afin de préparer des médicaments pour le traitement et/ou la prophylaxie de l'hypertonie, des cardiopathies coronariennes, des troubles circulatoires périphériques et cérébraux et/ou des maladies qui entraînent

une rétention d'eau ou de sodium accrue.